# EUROPEAN PATENT SPECIFICATION

(11) **EP 3 992 204 B1**
(45) Date of publication and mention of the grant of the patent: **18.11.2026**
(21) Application number: 20830547.4
(22) Date of filing: 29.06.2020
(51) Int. Cl.: C07K 14/74, C12N 5/10, C12N 5/0783

(54) **CELL FOR RESISTING TRANSPLANT REACTION AND METHOD**
ZELLE FÜR RESISTENZ GEGENÜBER TRANSPLANTATREAKTION UND VERFAHREN
CELLULE POUR RÉSISTER À UNE RÉACTION DE GREFFE ET PROCÉDÉ

(30) Priority: 29.08.2019 CN 201910809425
(43) Date of publication of application: 04.05.2022
(73) Proprietor: Carsgen Life Sciences Co., Ltd., Shanghai 200131 (CN)
(72) Inventor: LI, Zonghai, Shanghai 200231 (CN); LIAO, Zhaohui, Shanghai 200231 (CN)
(74) Representative: Jones Day
(86) International application number: PCT/CN2020/098930
(87) International publication number: WO 2020/259707

(56) References cited:
- WO-A1-2017/184901
- WO-A1-2018/183293
- CN-A- 106 103 475
- CN-A- 107 746 831
- CN-A- 107 828 730
- CN-A- 107 828 730
- CN-A- 108 341 872
- CN-A- 109 694 854
- US-A1- 2017 016 025
- DENG XINNA ET AL: "Antitumor Activity of NKG2D CAR-T Cells Against Human Colorectal Cancer Cells in Vitro and in Vivo", AMERICAN JOURNAL OF CANCER RESEARCH, vol. 9, no. 5, 15 May 2019 (2019-05-15), US, pages 945 - 958, XP055773925, ISSN: 2156-6976
- SANBER KHALED ET AL: "Graft- versus -host disease risk after chimeric antigen receptor T-cell therapy: the diametric opposition of T cells", vol. 195, no. 5, 25 May 2021 (2021-05-25), Hoboken, USA, pages 660 - 668, XP093073886, ISSN: 0007-1048, Retrieved from the Internet <URL:https://onlinelibrary.wiley.com/doi/full-xml/10.1111/bjh.17544> DOI: 10.1111/bjh.17544
- MO FEIYAN ET AL: "Engineered off-the-shelf therapeutic T cells resist host immune rejection", NATURE BIOTECHNOLOGY, vol. 39, no. 1, 3 February 2021 (2021-02-03), pages 56 - 63, XP037333517, ISSN: 1087-0156, DOI: 10.1038/S41587-020-0601-5
- RODRIGUEZ-BARBOSA J.I. ET AL: "Therapeutic implications of NK cell regulation of allogeneic CD8 T cell-mediated immune responses stimulated through the direct pathway of antigen presentation in transplantation", MABS, 23 July 2018 (2018-07-23), US, pages 1 - 15, XP093224252, ISSN: 1942-0862, DOI: 10.1080/19420862.2018.1502127
- GARNET SUCK; YEH CHING LINN; TORSTEN TONN: "Natural Killer Cells for Therapy of Leukemia", TRANSFUSION MEDICINE HEMOTHERAPY, vol. 43, no. 2, 1 January 2016 (2016-01-01), pages 89 - 95, XP055436764, ISSN: 1660-3796, DOI: 10.1159/000445325
- KATAYOUN REZVANI; RAYNE ROUCE; ENLI LIU; ELIZABETH SHPALL: "Engineering Natural Killer Cells for Cancer Immunotherapy", MOLECULAR THERAPY, vol. 25, no. 8, 31 August 2017 (2017-08-31), pages 1769 - 7181, XP055601573, ISSN: 1525-0016, DOI: 10.1016/j.ymthe.2017.06.012
- DENG XINNA, GAO FEI, LI NAN, LI QINGXIA, ZHOU YE, YANG TAO, CAI ZIQI, DU PINGPING, CHEN FAN, CAI JIANHUI: "Antitumor Activity of NKG2D CAR-T Cells Against Human Colorectal Cancer Cells in Vitro and in Vivo", AMERICAN JOURNAL OF CANCER RESEARCH, vol. 9, no. 5, 15 May 2019 (2019-05-15), pages 1 - 14, XP055773925, ISSN: 2156-6976

## Description

### FIELD OF THE INVENTION

The invention relates to a cell with the function of resisting transplantation rejection, and also relates to a method for resisting transplantation immune rejection, in particular to a method for resisting NK cell immune rejection.

### BACKGROUND OF THE INVENTION

Due to the immunogenetic differences between the donor and the recipient, when a graft from a donor is transplanted as an exogenous graft, the graft from the donor may also be recognized and attacked by immune cells in the recipient, thereby inhibiting or eliminating the exogenous graft and resulting in a host versus graft reaction (HVGR). The rejection reaction of the host's T cells to the graft may be effectively resisted by knocking out the MHC molecules in the graft cells, but it may cause rejection reaction of other immune cells in the host. For example, in allogeneic cell transplantation, when the MHC-I molecules of allogeneic cells are missing, it will cause rejection reaction of NK cells in the host, thereby enhancing the elimination of allogeneic cells (Nat. Biotechnol. 2017; 35(8): 765-772. doi:10.1038/nbt.3860). Therefore, how to effectively prevent the immune rejection of NK cells in a host is crucial to the development of allogeneic cell transplantation therapy. US 2017/016025 A1 describes a T cell expressing a soluble NKG2D ligand polypeptide. CN 109694854 A relates to universal CAR T cell in which the TCR expression is silenced.

### SUMMARY OF THE INVENTION

The object of the present invention is to provide a cell resistant to transplantation immune rejection, and a method for resisting transplantation immune rejection.

The technical solutions provided by the present invention include:
In the first aspect according to the present invention, provided herein is a T cell resistant to transplantation immune rejection, wherein the T cell expresses a first protein that is a chimeric receptor recognizing NKG2A on NK cells of a host; wherein the chimeric receptor comprises an extracellular domain having an antigen binding region recognizing NKG2A, a transmembrane domain, and an intracellular signaling domain; and wherein in the T cell the HLA-I gene and the endogenous TCR gene are silenced.

In some embodiments, the chimeric receptor is a chimeric antigen receptor (CAR), a chimeric T cell receptor, or a T cell antigen coupler (TAC).

In some embodiments, the antigen binding region is selected from the group consisting of scFv, Fv, Fab, Fab', Fab'-SH, F(ab')2, single domain fragment, and natural ligand engaging its homologous receptor.

In some embodiments, the first protein comprises an antigen binding region comprising HCDR1 sequence of SEQ ID NO: 10, HCDR2 sequence of SEQ ID NO: 11, HCDR3 sequence of SEQ ID NO: 12; and LCDR1 sequence of SEQ ID NO: 13, LCDR2 sequence of SEQ ID NO: 14, LCDR3 sequence of SEQ ID NO: 15.

In some embodiments, the first protein comprises an antigen binding region comprising a heavy chain variable region sequence of SEQ ID NO:1, or a light chain variable region sequence of SEQ ID NO: 2.

In some embodiments, the amino acid sequence of the first protein is represented by SEQ ID NO: 5.

In some embodiments, the HLA-I gene is one or more genes selected from the group consisting of: HLA-A, HLA-B, HLA-C, and B2M. In a specific embodiment, the HLA-I gene is B2M.

In some embodiments, the HLA-I gene and the endogenous TCR gene are silenced by gene editing technology.

In some embodiments, the gene editing technology is selected from the group consisting of: CRISPR/Cas9 technology, artificial zinc finger nuclease (ZFN) technology, transcription activator-like effector (TALE) technology, and TALE-CRISPR/Cas9 technology. In a specific embodiment, the gene editing technology is CRISPR/Cas9 technology. In a specific embodiment, the HLA-1 gene is silenced using a sgRNA comprising the sequence of SEQ ID NO: 8, and/or the TCR gene is silenced using a sgRNA comprising the sequence of SEQ ID NO: 7.

In some embodiments, the first protein further comprises an antigen binding region recognizing a tumor antigen or a pathogen antigen.

In some embodiments, the antigen binding region recognizing NKG2A of the host and the antigen binding region recognizing a tumor antigen or a pathogen antigen are connected by a linker peptide. In some embodiments, the first protein comprises the amino acid sequence of SEQ ID NO: 9.

In some embodiments, the T cell further expresses a second protein recognizing a tumor antigen or a pathogen antigen.

In some embodiments, the second protein is a chimeric receptor.

In some embodiments, the chimeric receptor is a CAR, a chimeric T cell receptor, or a TAC. In some embodiments, the second protein comprises an extracellular domain, a transmembrane domain, and an intracellular signaling domain.

In some embodiments, the second protein comprises an antigen binding region specifically recognizing BCMA. In a specific embodiment, the antigen binding region specifically recognizing BCMA comprises HCDR1 sequence of SEQ ID NO: 16, HCDR2 sequence of SEQ ID NO: 17, HCDR3 sequence of SEQ ID NO: 18, and LCDR1 sequence of SEQ ID NO: 19, LCDR2 sequence of SEQ ID NO: 20, LCDR3 sequence of SEQ ID NO: 21. In another embodiment, the antigen binding region specifically recognizing BCMA comprises a heavy chain variable region sequence of SEQ ID NO: 22 and a light chain variable region sequence of SEQ ID NO: 23. In specific embodiments, the amino acid sequence of the second protein is represented by SEQ ID NO: 6.

In some embodiments, the tumor antigen comprises thyroid stimulating hormone receptor (TSHR); CD171; CS-1; C-type lectin-like molecule-1; ganglioside GD3; Tn antigen; CD19; CD20; CD 22; CD 30; CD70; CD123; CD138; CD33; CD44; CD44v7/8; CD38; CD44v6; B7H3(CD276), B7H6; KIT(CD 117); interleukin 13 receptor subunit a (IL-13Ra); interleukin 11 receptor a (IL-1 lRa); prostate stem cell antigen (PSCA); prostate specific membrane antigen (PSMA); carcinoembryonic antigen (CEA); NY-ESO-1; HIV-l Gag; MART-1; gp100; tyrosinase; mesothelin; EpCAM; protease serine 21 (PRSS21); vascular endothelial growth factor receptor, vascular endothelial growth factor receptor 2 (VEGFR2); Lewis (Y) antigen; CD24; platelet derived growth factor receptor β (PDGFR-β); stage-specific embryonic antigen-4 (SSEA-4); cell surface-associated mucin 1 (MUCl), MUC6; epidermal growth factor receptor family and its mutants (EGFR, EGFR2, ERBB3, ERBB4, EGFRvIII); neural cell adhesion molecule (NCAM); carbonic anhydrase IX (CAIX); LMP2; ephrin A receptor 2 (EphA2); fucosyl GMl; sialyl Lewis adhesion molecule (sLe); ganglioside GM3; TGS5; high molecular weight melanoma-associated antigen (HMWMAA); O-acetyl GD2 ganglioside (OAcGD2); folic acid receptor; tumor vascular endothelial marker 1 (TEM1/CD248); tumor vascular endothelial marker 7 related (TEM7R); Claudin 6, Claudin 18.2, Claudin 18.1; ASGPRI; CDH16; 5T4; 8H9; αvβ6 integrin; B cell maturation antigen (BCMA); CA9; 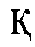 light chain (kappa light chain); CSPG4; EGP2, EGP40; FAP; FAR; FBP; embryonic AchR; HLA-Al, HLA-A2; MAGEAl, MAGE3; KDR; MCSP; NKG2D ligand; PSC1; ROR1; Sp17; SURVIVIN; TAG72; TEMl; conectin; tenascin; carcinoembryonic variant of tumor necrosis zone; G protein-coupled receptor C group 5-member D (GPRC5D); X chromosome open reading frame 61 (CXORF61); CD97; CD179a; anaplastic lymphoma kinase (ALK); polysialic acid; placenta-specific 1 (PLACl); hexose moiety of globoH glycoceramide (GloboH); breast differentiation antigen (NY-BR-1); uroplakin 2 (UPK2); hepatitis A virus cell receptor 1 (HAVCRI); adrenergic receptor β3 (ADRB3); pannexin 3 (PANX3); G protein-coupled receptor 20 (GPR20); lymphocyte antigen 6 complex locus K9 (LY6K); olfactory receptor 51E2 (OR51E2); TCRy alternating reading frame protein (TARP); Wilms tumor protein (WTl); ETS translocation variant gene 6 (ETV6-AML); sperm protein 17 (SPA17); X antigen family member lA (XAGE1); angiogenin binding cell surface receptor 2 (Tie2); melanoma cancer-testis antigen-1 (MAD-CT-1); melanoma cancer-testis antigen-2 (MAD-CT-2); Fas-related antigen 1; p53 mutant; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosaminyl transferase V (NA17); paired box protein Pax-3 (PAX3); androgen receptor; cyclin Bl; V-myc avian myeloidosis virus oncogene neuroblastoma-derived homolog (MYCN); Ras homolog family member C (RhoC); cytochrome P450 lBl (CYPlBl); CCCTC binding factor (zinc finger protein)-like (BORIS); squamous cell carcinoma antigen 3 (SART3) recognized by T cells; paired box protein Pax-5 (PAX5); proacrosin binding protein sp32 (OYTES 1); lymphocyte-specific protein tyrosine kinase (LCK); A kinase anchoring protein 4 (AKAP-4 ); synovial sarcoma X breakpoint 2 (SSX2); CD79a; CD79b; CD72; leukocyte-associated immunoglobulin-like receptor 1 (LAIR1); Fe fragment of IgA receptor (FCAR); leukocyte immunoglobulin-like receptor subfamily member 2 (LILRA2); CD300 molecular-like family member f (CD300LF); C-type lectin domain family 12 member A (CLEC12A); bone marrow stromal cell antigen 2 (BST2); containing EGF-like module mucin-like hormone receptor like 2 (EMR2); lymphocyte antigen 75 (LY75); glypican-3 (GPC3); Fc receptor-like 5 (FCRL5); immunoglobulin lambda-like polypeptide 1 (IGLL1).

In some embodiments, the pathogen antigen is selected from a group consisting of a virus antigen, a bacteria antigen, a fungus antigen, a protozoa antigen, and a parasite antigen. In a specific embodiment, the virus antigen is selected from a group consisting of a cytomegalovirus antigen, an Epstein-Barr virus antigen, a human immunodeficiency virus antigen, and an influenza virus antigen.

In some embodiments, the HLA-1 gene is silenced using a sgRNA, the sequence of which is represented by SEQ ID NO: 8, and/or the TCR gene is silenced using a sgRNA, the sequence of which is represented by SEQ ID NO: 7.

In some embodiments, the T cell is an autologous T cell or an allogeneic T cell.

In the second aspect according to the present invention, provided herein is a T cell of the invention for use in a method for preventing or regulating transplantation immune rejection.

In the third aspect according to the present invention, provided herein is a T cell of the invention for use in a method for preventing or regulating the attack of NK cell on exogenous cells.

In some embodiments, the exogenous cells are T cells, NK T cells, or stem cells; or engineered T cells, NK T cells, or stem cells.

In some embodiments, the T cells are administered before, after, or simultaneously with administration of the exogenous cells.

In a preferred embodiment, the exogenous cell is an immune effector cell; and preferably, the exogenous cell expresses a second receptor.

In a preferred embodiment, the second receptor is a chimeric receptor or a T cell receptor;
preferably, the chimeric receptor is selected from the group consisting of: a chimeric antigen receptor (CAR), a chimeric T cell receptor, and a T cell antigen coupler (TAC).

In a preferred embodiment, the second protein is a chimeric receptor, and the chimeric receptor is selected from the group consisting of: a chimeric antigen receptor (CAR), a chimeric T cell receptor, and a T cell antigen coupler (TAC); the chimeric receptor comprising a second protein comprises a second protein, a transmembrane domain, and an intracellular domain;
preferably, the second protein specifically recognizes BCMA or CD19;
preferably, the second protein comprises an antibody specifically recognizing BCMA;
more preferably, the antibody that specifically recognizes BCMA comprises HCDR1 represented by SEQ ID NO: 16, HCDR2 represented by SEQ ID NO: 17, HCDR3 represented by SEQ ID NO: 18, and LCDR1 represented by SEQ ID NO: 19, LCDR2 represented by SEQ ID NO: 20, LCDR3 represented by SEQ ID NO: 21;
more preferably, the antibody specifically recognizing BCMA comprises a heavy chain variable region represented by SEQ ID NO: 22 and a light chain variable region represented by SEQ ID NO: 23.

### BRIEF DESCRIPTION OF THE DRAWINGS

Fig. 1: Expression of NK cell surface markers;
Fig. 2: Expression of NK cell surface markers in T cells;
Fig. 3: Growth characteristics of NKG2A CAR-T cells; panel A, cell proliferation curve; panel B, cell diameter; panel C, CAR positive rate;
Fig. 4: In vitro killing ability of NKG2A CAR-T cells on NK cells after a total of 4 hours of incubation;
Fig. 5: In vitro killing ability of NKG2A CAR-T cells on NK cells after a total of 18 hours of incubation;
Fig. 6: Efficiently knocking out TCR and B2M in CAR-T cells;
Fig. 7A, Fig. 7B, Fig. 7C and Fig. 7D: The resistance of NKG2A UCAR-T cells to NK cells detected by FACS;
Fig. 8: The plasmid map of CAR targeting NKG2A;
Fig. 9: The plasmid map of CAR targeting BCMA;
Fig. 10: The resistance of NKG2A UCAR-T cells to NK cells detected by FACS;
Fig. 11: UCAR-T cell survival in peripheral blood of mice detected by FACS;
Fig. 12: The schematic diagram of the structure of BCMA-GS-NKG2A UCAR-T;
Fig. 13: Preparation of BCMA-GS-NKG2A UCAR-T cells;
Fig. 14: In vitro anti-tumor effect of BCMA-GS-NKG2A UCAR-T cells;
Fig. 15: The resistance of BCMA-GS-NKG2A UCAR-T cells to NK cells;
Fig. 16: The plasmid map of PRRL-BCMA-BBZ-F2A-EGFP;
Fig. 17: The plasmid map of PRRL-NKG2A-28Z-F2A-EGFP;
Fig. 18: The plasmid map of PRRL-BCMA-GS-NKG2A-BBZ;
Fig. 19: The results of in vivo resistance of BCMA-GS-NKG2A UCAR-T cells to NK cells.

### DETAIL DESCRIPTION OF THE INVENTION

Unless specifically defined, all technical and scientific terms used herein have the same meaning as commonly understood by those skilled in the fields of gene therapy, biochemistry, genetics, and molecular biology. All methods and materials similar or equivalent to those described herein can be used in the practice or testing of the present invention, in which suitable methods and materials are described herein. In case of conflict, the specification including definitions will control. In addition, unless otherwise specified, the materials, methods, and examples are illustrative only and not intended to be limiting.

Unless otherwise specified, the practice of the present invention will adopt the traditional techniques of cell biology, cell culture, molecular biology, transgenic biology, microbiology, recombinant DNA and immunology, which all belong to the technical scope of this field. These techniques are fully explained in the references. See, for example, Current Protocols in Molecular Biology (Frederick M. AUSUBEL, 2000, Wiley and Son Inc, Library of Congress, USA); Molecular Cloning: A Laboratory Manual, Third Edition, (Sambrook Harbor, 2001, Cold Spring Harbor, New York: Cold Spring Harbor Laboratory Press); Oligonucleotide Synthesis (M. J. Gaited., 1984); Mullis et al. U.S. Pat. No. 4,683,195; Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In Enzymology (J. Abelson and M. Simon, eds.-in-chief, Academic Press, Inc., New York), especially Vols.154 and 155 (Wu et al.eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Hand book Of Experimental Immunology, Volumes I-IV (D. M. Weir and C. C. Blackwell, eds., 1986); and Manipulating the Mouse Embryo (Cold Spring Harbor Laboratory Press, Cold Spring Harbor, NY, 1986).

In the description, all aspects of the claimed subject matter are presented in range format. It should be understood that, the description in range format is merely for convenience and brevity, and should not be construed as an inflexible limitation on the scope of the claimed subject matter. Therefore, the description of a range should be considered to have specifically disclosed all possible subranges as well as individual values within the range. For example, in the case of providing a range of values, it should be understood that each intermediate value between the upper limit and the lower limit of the range and any other stated or intermediate values within the range are included within the claimed subject matter, the upper and lower limits of the scope also belong to the scope of the claimed subject matter. The upper and lower limits of these smaller ranges may be independently included in such a smaller range, and they also belong to the scope of the claimed subject matter, unless the upper and lower limits of the range are explicitly excluded. When the set range includes one or two limit values, the claimed subject matter also includes a range that excludes one or two of the limit values. This applies regardless of the width of the range.

The term "about" used herein refers to the usual error range of each value easily known to those skilled in the art. When "about" is used herein before a value or parameter, an embodiment referring to the value or parameter itself is also included (and described). For example, description of "about X" includes description of "X". For example, "about" or "comprise" may refer to a value of standard deviation within 1 or more than 1 according to the actual situation in the field. Alternatively, "about" or "comprise" may refer to a range of up to 10% (i.e., 10%). For example, about 5 µM may include any value between 4.5 µM and 5.5 µM. When a specific value or composition is provided in the application and the scope of the patent application, unless otherwise indicated, "about" or "comprise" shall be assumed to be within the acceptable error range of the specific value or composition.

Unless otherwise indicated, any concentration range, percentage range, ratio range, or integer range described herein should be understood to include any integer within the stated range, and where appropriate, the value of its fraction (for example, one-tenth, and one-hundredth of an integer).

To facilitate a better understanding of the present invention, the related terms are defined as follows:
The term "transplantation immune rejection" refers to such an immunological response, in which after a graft, such as a heterologous tissue, organ, or cell is transplanted into a host, the exogenous graft as an "exogenous component" is recognized by the host's immune system, and the immune system initiates an attack on the graft and tries to destruct and remove it.

The term "graft" refers to a biological material or preparation derived from an individual other than the host and used to be implanted into the host. The graft may be derived from any animal source, such as a mammalian source, preferably from a human. In some embodiments, the graft may be derived from a host, for example, cells from the host are cultured in vitro or modified to be implanted into the host again. In some embodiments, the graft may be derived from another allogeneic individual, for example, cells from other people are cultured in vitro or modified to be implanted into the host. In some embodiments, the graft may be a heterogeneous individual, such as organs from other species (such as mice, pigs, and monkeys) to be implanted into humans.

The term "cell" and its other grammatical forms may refer to a cell derived from human or non-human animal.

The term "host" refers to a recipient who receives transplantation of a graft. In some embodiments, it may be an individual, such as a human, who receives transplantation of exogenous cells.

The term "immune effector cells" refers to cells that participate in immune responses and produce immune effects, such as T cells, B cells, natural killer (NK) cells, natural killer T (NK T) cells, dendritic cells, CIK cells, and macrophages, mast cells, etc. In some embodiments, the immune effector cells are T cells, NK cells, NK T cells. In some embodiments, the T cell may be an autologous T cell, a heterologous T cell, or an allogeneic T cell. In some embodiments, the NK cells may be allogeneic NK cells.

The term "artificially modified cell with the function of immune effector cell" refers to a cell or cell line without immune effect acquires the function of immune effector cell after being artificially modified or stimulated by a stimulus. For example, 293T cells are artificially modified to have the function of immune effector cells; for example, stem cells are induced in vitro to differentiate into immune effector cells.

In some cases, "T cells" may be pluripotent stem cells derived from bone marrow, which differentiate and mature into mature T cells with immunological activity in the thymus. In some cases, "T cells" may be cell populations with specific phenotypic characteristics, or mixed cell populations with different phenotypic characteristics, for example, "T cells" may be cells comprising at least one T cell subpopulation selected from the group consisting of: stem cell-like memory T cells (Tscm cells), central memory T cells (Tcm), effector T cells (Tef, Teff), regulatory T cells (tregs), and/or effector memory T cells (Tem). In some cases, "T cells" may be T cells of a specific subtype, such as γδ T cells.

T cells may be obtained from many sources, including PBMC, bone marrow, lymph node tissue, umbilical cord blood, thymus tissue, and tissue from infection sites, ascites, pleural effusion, spleen tissue, and tumors. In some cases, any number of techniques known to those skilled in the art, such as FicollTM isolation, may be used to obtain T cells from blood collected from an individual. In one embodiment, cells from the circulating blood of an individual are obtained by apheresis. Apheresis products usually comprise lymphocytes, including T cells, monocytes, granulocytes, B cells, other nucleated white blood cells, red blood cells and platelets. In one embodiment, the cells collected by apheresis collection may be washed to remove plasma molecules, then placing in a suitable buffer or medium for use in subsequent processing steps. Alternatively, cells may be derived from healthy donors, or patients diagnosed with cancer.

The term "MHC" refers to histocompatibility complex, which is a general term for all the gene groups encoding the antigens of the biocompatibility complex. MHC antigens are expressed in the tissues of all higher vertebrates, called HLA antigens in human cells, playing an important role in the transplant reaction; and rejection is mediated by T cells that respond to the histocompatibility antigen on the surface of the implanted tissue. MHC protein plays a vital role in T cell stimulation. Antigen presenting cells (usually dendritic cells) display peptides (which belong to MHC) as degradation products of exogenous proteins on the cell surface, in the presence of co-stimulatory signals, T cells are activated and act on target cells that also display the same peptide/MHC complex. For example, stimulated T helper cells will target macrophages displaying antigens bound to their MHC, or cytotoxic T cells (CTL) will act on virus-infected cells displaying exogenous viral peptides. MHC antigens are divided into MHC class I antigens and MHC class II antigens.

The term "human leukocyte antigen" (HLA) is the coding gene of the human major histocompatibility complex, located on chromosome 6 (6p21.31), and is closely related to the function of the human immune system. HLA comprises class I, class II and class III gene parts. The antigens expressed by HLA class I and class II genes are located on the cell membrane, and they are MHC-I (encoded by HLA-A, HLA-B, HLA-C site) and MHC-II (encoded by HLA-D region), HLA class I is almost distributed on the surface of all cells of the body, and is a heterodimer composed of heavy chain (α chain) and β2 microglobulin (B2M). HLA class II is mainly glycoprotein located on the surface of macrophages and B lymphocytes.

The term "B2M" refers to β-2 microglobulin, also known as B2M, which is the light chain of a MHC class I molecule. In humans, B2M is encoded by the b2m gene located on chromosome 15, as opposed to other MHC genes located as gene clusters on chromosome 6. Studies have shown that when the B2M gene is mutated, hematopoietic grafts from mice that lack MHC I expression on the surface of normal cells are rejected by NK cells in normal mice, indicating that the defective expression of MHC I molecules makes the cells vulnerable to rejection by the host immune system (Bix et al. 1991).

The term "chimeric receptor" refers to a fusion molecule formed by linking DNA fragments or cDNAs corresponding to proteins from different sources by gene recombination technology, comprising extracellular domain, transmembrane domain and intracellular domain. Chimeric receptors include but are not limited to: chimeric antigen receptor (CAR), chimeric T cell receptor (TCR), and T cell antigen coupler (TAC).

The term "chimeric antigen receptor" (CAR) comprises extracellular antigen binding domain, transmembrane domain, and intracellular signaling domain. Intracellular signaling domain comprises functional signaling domain of stimulatory molecule and/or costimulatory molecule. In one aspect, the stimulatory molecule is a ζ chain that binds to the T cell receptor complex; in one aspect, cytoplasmic signaling domain further comprises functional signaling domain of one or more costimulatory molecules, such as 4-1BB (i.e., CD137), CD27, and/or CD28.

The term "T cell receptor (TCR)" mediates the recognition of specific major histocompatibility complex (MHC)-restricted peptide antigens by T cells, including classic TCR receptor and optimized TCR receptor. The classic TCR receptor is composed of two peptide chains, α and β, wherein each peptide chain may be divided into variable region (V region), constant region (C region), transmembrane region, cytoplasmic region and the like; and the antigen specificity exists in the V region, wherein each of the V regions (Vα, Vβ) has three hypervariable regions, CDR1, CDR2, and CDR3. In one aspect, as for T cells expressing classic TCR, the specificity of the TCR of T cells to the target antigen may be induced by methods such as antigen stimulation on T cells.

The term "chimeric T cell receptor" comprises recombinant polypeptides derived from various polypeptides constituting the TCR, which can bind to the surface antigens of target cells and interact with other polypeptides of the complete TCR complex, usually co-localized in T cell surface. The chimeric T cell receptor is composed of a TCR subunit and an antigen binding domain comprising a human or humanized antibody domain, wherein the TCR subunit comprises at least part of the TCR extracellular domain, transmembrane domain, and stimulation domain of the intracellular signal domain of the TCR intracellular domain; the TCR subunit and the antibody domain are effectively connected, wherein the extracellular domain, transmembrane domain, and intracellular signal domain of the TCR subunit are derived from CD3ε or CD3γ, and the chimeric T cell receptor is integrated into the TCR expressed on the T cell.

The term "T cell antigen coupler (TAC)" comprises three functional domains: 1. the antigen binding domain, comprising single-chain antibodies, and designed ankyrin repeat proteins (DARPin), or other targeting groups; 2. the extracellular domain, which is a single-chain antibody binding to CD3, so that the TAC receptor and the TCR receptor are close to each other; 3. the transmembrane region and the intracellular region of the CD4 co-receptor, wherein the intracellular domain is connected to the protein kinase LCK to catalyze the phosphorylation of immunoreceptor tyrosine activation motifs (ITAMs) of the TCR complex as the initial step of T cell activation.

The term "signaling domain" refers to a functional part of a protein that functions by transmitting information in a cell, and is used to regulate the cell activity through a certain signaling pathway by generating a second messenger or acting as an effector in response to such a messenger. The intracellular signaling domain may comprise all intracellular parts of the molecule, or all natural intracellular signaling domains, or functional fragments or derivatives thereof.

The term "co-stimulatory molecule" refers to a molecule that binds to a cell stimulating signal molecule, such as TCR/CD3, and leads to the proliferation of T cells and/or the up- or down-regulation of key molecules.

The terms "activating" and "activation" are used interchangeably and may refer to the process by which a cell changes from a resting state to an active state. The process may comprise a response to antigen and a response to phenotypic or genetic changes in migration, and/or functional activity status. For example, the term "activation" may refer to the process of gradually activating T cells. For example, T cells may require at least one signal to be fully activated.

The term "gene editing" refers to the ability of humans in "editing" target genes, so as to achieve the knockout and addition of specific DNA fragments.

The term "gene silencing" refers to a phenomenon in which genes are not expressed or underexpressed due to various reasons. Gene silencing may be gene silencing at the transcriptional level caused by DNA methylation, heterochromatinization and positional effects, or post-transcriptional gene silencing, i.e., a gene is inactivated by specific inhibition of target RNA at the post-transcriptional level, including antisense RNA, co-suppression, gene suppression, RNA interference, and microRNA-mediated translational inhibition.

The "TCR silencing" means that the endogenous TCR is not expressed or is underexpressed.

The "MHC silencing" means that the endogenous MHC is not expressed or is underexpressed.

The term "CRISPR" refers to clustered regularly interspaced short palindromic repeats.

The term "Cas9" refers to a CRISPR-associated nuclease, which is related to an RNA-guided technology for editing targeted genes by using Cas9 nuclease.

The "CRISPR/Cas9 system" is collectively referred as transcripts and other elements involved in the expression of Cas9 enzyme genes or directing its activity, comprising a sequence encoding Cas9 gene, tracr (transactivation CRISPR) sequence (such as tracrRNA or active part of tracrRNA), tracr matching sequence (encompassing "direct repeat" and partial direct repeat processed by tracrRNA in the context of endogenous CRISPR system), guide sequence (also called "spacer", namely gRNA, in the context of endogenous CRISPR system), or other sequences and transcripts from the CRISPR locus.

The term "target sequence" refers to a sequence complementary to a guide sequence. The complementary pairing between the target sequence and the guide sequence promotes the formation of a CRISPR complex. A target sequence may comprise any polynucleotide, such as DNA or RNA polynucleotide. In some embodiments, the target sequence is located in the nucleus or cytoplasm of a cell.

Generally speaking, a guide sequence (gRNA) is any polynucleotide sequence that has sufficient complementarity with a target polynucleotide sequence, so as to hybridize with the target sequence and guide the sequence-specific binding of the CRISPR complex to the target sequence. In some embodiments, when a suitable alignment algorithm is used for optimal alignment, the degree of complementarity between the guide sequence and its corresponding target sequence is about or more than about 50%, 60%, 75%, 80%, 85%, 90%, 95%, 97.5%, 99%, or more. Any suitable algorithm for aligning sequences may be used to determine the best alignment, non-limiting examples of the algorithms include the Smith-Waterman algorithm, the Needleman-Wunsch algorithm, algorithms based on Burrows-Wheeler Transform (e.g., Burrows Wheeler Aligner), ClustalW, Clustal X, BLAT, Novoalign (Novocraft Technology Company), ELAND (Illumina Company, San Diego, California), SOAP (available at soap.genomics.org.cn), and Maq (available at maq.sourceforge.net).

In some embodiments, the CRISPR enzyme is part of a fusion protein comprising one or more heterologous protein domains (e.g., about or more than about 1, 2, 3, 4, 5, 6, 7, 8, 9, 10 or more domains other than the CRISPR enzyme). The CRISPR enzyme fusion protein may comprise any other protein, and optionally a linking sequence between any two domains. Examples of protein domains that may be fused to CRISPR enzymes include, but are not limited to, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include: histidine (His) tags, V5 tags, FLAG tags, influenza virus hemagglutinin (HA) tags, Myc tags, VSV-G tags, and thioredoxin (Trx) tags.

The term "Cas9 enzyme" may be wild-type Cas9, or artificially modified Cas9.

The term "sgRNA" refers to short gRNA.

During gene editing, the given gRNA, tracr pairing sequence, and tracr sequence may be given separately or as a complete RNA sequence.

The combination of Cas9 protein and gRNA may realize the cleavage of DNA at specific sites. The recognition sequence of CRISPR/Cas system derived from *Streptococcus pyogenes* is 23bp, and may target 20bp. The last 3 positions NGG sequence of the recognition site is called PAM (protospacer adjacent motif) sequence.

The Cas transgene may be delivered by vectors (e.g., AAV, adenovirus, lentivirus), and/or particles and/or nanoparticles, and/or electroporation.

In one embodiment, the exons of the corresponding coding genes in the constant regions of one or both of the α and β chains of the TCR are knocked out by CRISPER/Cas technology, so as to make the endogenous TCR inactive; preferably, the first exon of the constant region of the endogenous TCRα chain is targeted to be knocked out.

"Inhibiting" or "suppressing" the expression of B2M or TCR means that the expression of B2M or TCR in a cell is reduced by at least 1%, at least 5%, at least 10%, at least 20%, at least 30%, at least 40%, at least 50%, at least 60%, at least 70%, at least 80%, at least 90%, at least 95%, at least 99%, or 100%. More specifically, "inhibiting" or "suppressing" the expression of B2M means that the content of B2M in a cell is reduced by at least 1%, at least 5%, at least 10%, at least 20%, at least 30%, at least 40%, at least 50%, at least 60%, at least 70%, at least 80%, at least 90%, at least 95%, at least 99% or 100%. The expression or content of proteins in cells may be determined by any suitable method known in the art, such as ELISA, immunohistochemistry, Western blotting or flow cytometry, using B2M or TCR specific antibodies.

The term "modification" used herein refers to a change in the state or structure of the protein or polypeptide according to the present invention. Modification methods may be chemical, structural and functional modification.

The term "transfection" refers to the introduction of exogenous nucleic acid into eukaryotic cells. Transfection may be achieved by various means known in the art, including calcium phosphate-DNA co-precipitation, DEAE-dextran-mediated transfection, polybrene-mediated transfection, electroporation, microinjection, liposome fusion, lipofection, protoplast fusion, retroviral infection and biolistics.

The terms "encoding nucleic acid molecule", "encoding DNA sequence" and "encoding DNA" refer to the sequence or order of deoxyribonucleotides along a deoxyribonucleic acid chain. The order of these deoxyribonucleotides determines the order of amino acids along the polypeptide (protein) chain. Therefore, the nucleic acid sequence encodes an amino acid sequence.

The term "individual" refers to any animal, such as a mammal or marsupial. Individuals of the present invention include, but are not limited to, humans, non-human primates (such as rhesus monkeys or other types of macaques), mice, pigs, horses, donkeys, cattle, sheep, rats, and any kind of poultry.

The term "peripheral blood mononuclear cells" (PBMCs) refers to cells with mononuclear nuclei in peripheral blood, comprising lymphocytes and monocytes.

The term "T cell activating" or "T cell activation" and other grammatically other forms may refer to the state of T cells that are sufficiently stimulated to induce detectable cell proliferation, cytokine production, and/or detectable effector function.

When being used to refer to a nucleotide sequence, the term "sequence" and other grammatical forms as used herein may include DNA or RNA, and may be single-stranded or double-stranded.

The term "effective amount" as used herein refers to an amount that provides a therapeutic or preventive benefit.

The term "expression vector" as used herein refers to a vector comprising a recombinant polynucleotide, which comprises an expression control sequence operatively linked to the nucleotide sequence to be expressed. The expression vector comprises sufficient cis-acting elements for expression; other elements for expression may be provided by host cells or in vitro expression systems. Expression vectors include all those known in the art, such as plasmids, viruses (e.g., lentivirus, retrovirus, adenovirus, and adeno-associated virus).

The term "vector" as used herein is a composition that comprises an isolated nucleic acid and may be used to deliver the isolated nucleic acid into a cell. Many vectors are known in the art, including but not limited to: linear polynucleotides, polynucleotides related to ionic or amphiphilic compounds, plasmids, and viruses. Therefore, the term "vector" comprises autonomously replicating plasmids or viruses, and may also comprise non-plasmid and non-viral compounds that promote the transfer of nucleic acid into cells, such as polylysine compounds, liposomes, and the like.

As used herein, the term sequence "identity" means the percent identity determined by comparing two best-matched sequences over a comparison window (for example, at least 20 positions), wherein the portion of the polynucleotide or polypeptide sequence in the comparison window may comprise additions or deletions (i.e., gaps), for example, 20% or less gaps (e.g., 5-15%, or 10-12%) as compared to the reference sequence (which does not comprise additions or deletions) for the two best-matched sequences. The percentage is usually calculated by determining the number of positions where the same nucleic acid bases or amino acid residues occur in the two sequences, so as to produce the number of correct matching positions. The number of correct matching positions is divided by the total number of positions in the reference sequence (i.e., the window size), and multiply the result by 100 to produce the percentage of sequence identity.

The term "exogenous" as used herein refers to a nucleic acid molecule or polypeptide, cell, tissue, etc. which is not endogenously expressed in the organism itself, or the expression level is insufficient to achieve the function when it is overexpressed.

The term "endogenous" refers to a nucleic acid molecule or polypeptide derived from the organism itself.

In some embodiments, the chimeric receptor according to the present invention is a chimeric antigen receptor.

The chimeric antigen receptor usually comprises an extracellular antigen binding region. In some embodiments, the extracellular antigen binding region may be fully human, humanized, or murine; alternatively, the chimera in the extracellular antigen binding region comprises amino acid sequences from at least two different animals.

Examples of extracellular antigen binding regions may be scFv, Fv, Fab, Fab', Fab'-SH, F(ab')2, single domain fragment, or natural ligand engaging its homologous receptor, and any derivative thereof.

In some aspects, the extracellular antigen binding region (e.g., scFv) may comprise an antigen-specific light chain CDR. In some cases, a light chain CDR may comprise two or more light chain CDRs, which may be referred as light chain CDR-1, CDR-2, and the like. In some cases, the light chain CDR may comprise three light chain CDRs, which may be referred as light chain CDR-1, light chain CDR-2, and light chain CDR-3, respectively. In one embodiment, a group of CDRs present on a common light chain may be collectively referred as light chain CDRs.

In some aspects, the extracellular antigen binding region (e.g., scFv) may comprise an antigen-specific heavy chain CDR. The heavy chain CDR may be an antigen binding unit, such as the heavy chain complementarity determining region of a scFv. In some cases, a heavy chain CDR may comprise two or more heavy chain CDRs, which may be referred as heavy chain CDR-1, CDR-2, and the like. In some cases, the heavy chain CDR may comprise three heavy chain CDRs, which may be referred as heavy chain CDR-1, heavy chain CDR-2, and heavy chain CDR-3, respectively. In one embodiment, a group of CDRs present on a common heavy chain may be collectively referred as a heavy chain CDR.

The extracellular antigen binding region may be modified in various ways by genetic engineering. In some cases, the extracellular antigen binding region may be mutated, so that the extracellular antigen binding region may be selected to have a higher affinity for its target. In some cases, the affinity of the extracellular antigen binding region for its target may be optimized for targets expressed at low levels on normal tissues. This optimization may be done to minimize potential toxicity. In other cases, clones of extracellular antigen binding regions with higher affinity for the membrane-bound form of the target may be superior to their soluble form counterparts. This modification may be made, because different levels of targets in soluble form may also be detected, and their targeting may cause undesirable toxicity.

In some cases, the extracellular antigen binding region also comprises hinges or spacers, and the terms "hinge" and "spacer" may be used interchangeably. The hinge may be considered as part of the CAR used to provide flexibility to the extracellular antigen binding region. For example, the hinge may be the natural hinge region of the CD8α molecule.

The term "transmembrane domain" refers to a domain which may anchor the chimeric protein to the plasma membrane of a cell. For example, the transmembrane domains of CD28 and CD8α may be used.

The term "regulation" refers to positive or negative changes. Examples of regulation comprise 1%, 2%, 10%, 25%, 50%, 75%, or 100% changes. In a specific embodiment, it refers to a negative change.

The term "treatment" refers to interventions that are trying to change the course of the disease, either for prevention or intervention in the clinical pathological process. The therapeutic effect includes, but is not limited to: preventing the occurrence or recurrence of a disease, reducing the symptoms, reducing the direct or indirect pathological consequences of any disease, preventing metastasis, slowing the progression of a disease, improving or relieving the condition, relieving or improving the prognosis, etc.

The term "prevention" refers to an intervention conducting before the occurrence of a disease (such as rejection caused by cell transplantation).

The first protein according to the present invention refers to the above-mentioned protein recognizing one or more immune effector cells of the host.

The second protein according to the present invention refers to the above-mentioned protein recognizing tumor antigens or pathogen antigens.

The "second receptor" and "protein recognizing one or more immune effector cells of the host" according to the present invention may be expressed in tandem, or expressed separately.

When the "second receptor" and the "protein recognizing one or more immune effector cells of the host" are expressed separately, they have independent transmembrane domains and intracellular domains. For the expression method, please refer to WO 2016/086813 A1, Enhancing the specificity of T-cell cultures for adoptive immunotherapy of cancer, Duong CP et al., Immnuotherapy 3(1): 33-48, etc.

When the "second receptor" of the present invention is expressed in tandem with the "protein recognizing one or more immune effector cells of the host", the protein recognizing one or more immune effector cells of the host may also recognize the antigens recognized by the "second receptor", such as tumor antigens.

"Tumor antigen" refers to an antigen newly emerged or overexpressed during the occurrence and development of hyperproliferative diseases. In certain aspects, the hyperproliferative disorder according to the present invention refers to cancer.

The tumor antigens according to the present invention may be solid tumor antigens, or hematological tumor antigens.

The tumor antigens according to the present invention include but are not limited to: thyroid stimulating hormone receptor (TSHR); CD171; CS-1; C-type lectin-like molecule-1; ganglioside GD3; Tn antigen; CD19; CD20; CD 22; CD 30; CD 70; CD 123; CD 138; CD33; CD44; CD44v7/8; CD38; CD44v6; B7H3(CD276), B7H6; KIT(CD117); interleukin 13 receptor subunit α (IL-13Rα); interleukin 11 receptor α (IL-1IRα); prostate stem cell antigen (PSCA); prostate specific membrane antigen (PSMA); carcinoembryonic antigen (CEA); NY-ESO-1; HIV-1Gag; MART-1; gp100; tyrosinase; mesothelin; EpCAM; protease serine 21 (PRSS21); vascular endothelial growth factor receptor, vascular endothelial growth factor receptor 2 (VEGFR2); Lewis (Y) antigen; CD24; platelet derived growth factor receptor β (PDGFR-β); stage-specific embryonic antigen-4 (SSEA-4); cell surface-associated mucin 1 (MUC1), MUC6; epidermal growth factor receptor family and its mutants (EGFR, EGFR2, ERBB3, ERBB4, EGFRvIII); neural cell adhesion molecule (NCAM); carbonic anhydrase IX (CAIX); LMP2; ephrin A receptor 2 (EphA2); fucosyl GM1; sialyl Lewis adhesion molecule (sLe); ganglioside GM3; TGS5; high molecular weight melanoma-associated antigen (HMWMAA); O-acetyl GD2 ganglioside (OAcGD2); folic acid receptor; tumor vascular endothelial marker 1 (TEM1/CD248); tumor vascular endothelial marker 7 related (TEM7R); Claudin 6, Claudin 18.2, Claudin 18.1; ASGPR1; CDH16; 5T4; 8H9; αvβ6 integrin; B cell maturation antigen (BCMA); CA9; κ light chain (kappa light chain); CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; conectin; tenascin; carcinoembryonic variant of tumor necrosis zone; G protein-coupled receptor C group 5-member D (GPRC5D); X chromosome open reading frame 61 (CXORF61); CD97; CD179a; anaplastic lymphoma kinase (ALK); polysialic acid; placenta-specific 1 (PLAC1); hexose moiety of globoH glycoceramide (GloboH); breast differentiation antigen (NY-BR-1); uroplakin 2 (UPK2); hepatitis A virus cell receptor 1 (HAVCR1); adrenergic receptor β3 (ADRB3); pannexin 3 (PANX3); G protein-coupled receptor 20 (GPR20); lymphocyte antigen 6 complex locus K9 (LY6K); olfactory receptor 51E2 (OR51E2); TCRγ alternating reading frame protein (TARP); Wilms tumor protein (WT1); ETS translocation variant gene 6 (ETV6-AML); sperm protein 17 (SPA17); X antigen family member 1A (XAGE1); angiogenin binding cell surface receptor 2 (Tie2); melanoma cancer-testis antigen-1 (MAD-CT-1); melanoma cancer-testis antigen-2 (MAD-CT-2); Fos-related antigen 1; p53 mutant; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosaminyl transferase V (NA17); paired box protein Pax-3 (PAX3); androgen receptor; cyclin B1; V-myc avian myeloidosis virus oncogene neuroblastoma-derived homolog (MYCN); Ras homolog family member C (RhoC); cytochrome P450 1B1 (CYP1B1); CCCTC binding factor (zinc finger protein)-like (BORIS); squamous cell carcinoma antigen 3 (SART3) recognized by T cells; paired box protein Pax-5 (PAX5); proacrosin binding protein sp32 (OYTES1); lymphocyte-specific protein tyrosine kinase (LCK); A kinase anchoring protein 4 (AKAP-4); synovial sarcoma X breakpoint 2 (SSX2); CD79a; CD79b; CD72; leukocyte-associated immunoglobulin-like receptor 1 (LAIR1); Fc fragment of IgA receptor (FCAR); leukocyte immunoglobulin-like receptor subfamily member 2 (LILRA2); CD300 molecular-like family member f (CD300LF); C-type lectin domain family 12 member A (CLEC12A); bone marrow stromal cell antigen 2 (BST2); containing EGF-like module mucin-like hormone receptor like 2 (EMR2); lymphocyte antigen 75 (LY75); glypican-3 (GPC3); Fc receptor-like 5 (FCRL5); immunoglobulin lambda-like polypeptide 1 (IGLL1). Preferably, the tumor antigen is BCMA or CD19.

The pathogen antigen is selected from the group consisting of: virus, bacteria, fungus, protozoa, and parasite antigen; the virus antigen is selected from the group consisting of: cytomegalovirus antigen, Epstein-Barr virus antigen, human immunodeficiency virus antigen, and influenza virus antigen.

The present invention will be further explained below in conjunction with specific examples. The following examples do not specify the experimental methods of specific conditions, usually in accordance with conventional conditions described in Molecular Cloning Experiment Guide, Edited by J. Sambrook et al., the Third Edition, Science Press, 2002, or according to the conditions recommended by the manufacturer.

### Example 1: Detection of the Expression of NK Cell Surface Receptor

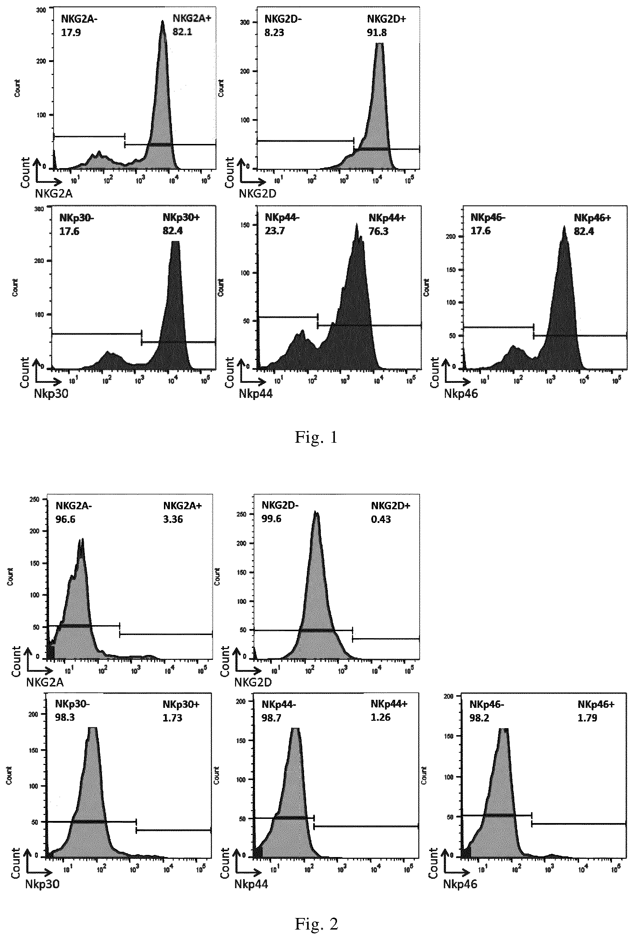

Using Ficoll-Paque (GE bioscience) for density gradient centrifugation, monocytes are separated from peripheral blood; and negative screening is performed with NK cell separation kit (purchased from Miltenyi), after removing T cells, B cells, and monocytes, etc., cell phenotype identification and expansion are performed in vitro. The receptors on the surface of the isolated NK cells, such as NKG2A, NKG2D, NKP30, NKP44, NKP46 and other markers are identified by flow cytometry. Flow cytometry results show that NKG2A, NKP30, NKP44, and NKP46 are expressed in about 80% of the NK cells, while NKG2D is expressed in more than 90% of the NK cells (see Fig. 1).

Furthermore, we also tested the expression of the above-mentioned surface markers in T cells. T cells activated by CD3/CD28 magnetic beads (purchased from Thermo Fisher) are collected, culturing to day 8 for flow cytometry. Experimental results show that NKG2A, NKG2D, NKP30, NKP44, and NKP46 are almost not expressed in T cells, indicating that the above-mentioned markers may be used as targets for NK cells (see Fig. 2).

### Example 2: Preparation and Functional Verification of CAR-T Cells

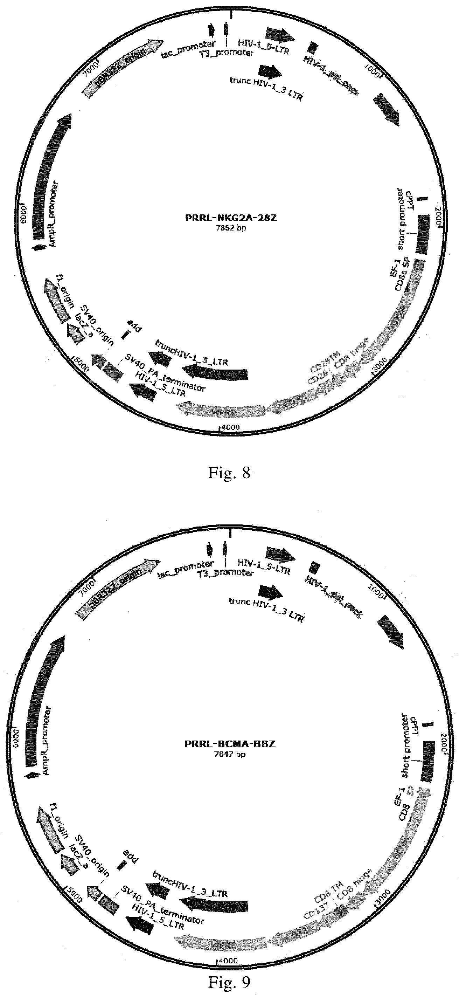

1. NKG2A is selected as a representative target to prepare CAR-T cells targeting NK cells. Referring to conventional operations to design and construct a vector of the chimeric antigen receptor (the amino acid sequence is represented by SEQ ID NO: 5) comprising anti-NKG2A single-chain antibody (the amino acid sequence of VH is represented by SEQ ID NO: 1, and the amino acid sequence of VL is represented by SEQ ID NO: 2), CD28 transmembrane domain and intracellular domain (the amino acid sequence is represented by SEQ ID NO: 3), T cell activating factor CD3ζ (the amino acid sequence is represented by SEQ ID NO: 4), the plasmid map is shown in Fig. 8. The plasmid is packaged by lentivirus, and named as VRRL-NKG2A-28Z(TM).

After 48 hours of T cell activation and expansion, the cell density is adjusted to 2*10^6/mL, and VRRL-NKG2A-28Z(TM) lentivirus is added at the ratio of MOI=10 to obtain CAR-T cells targeting NKG2A.

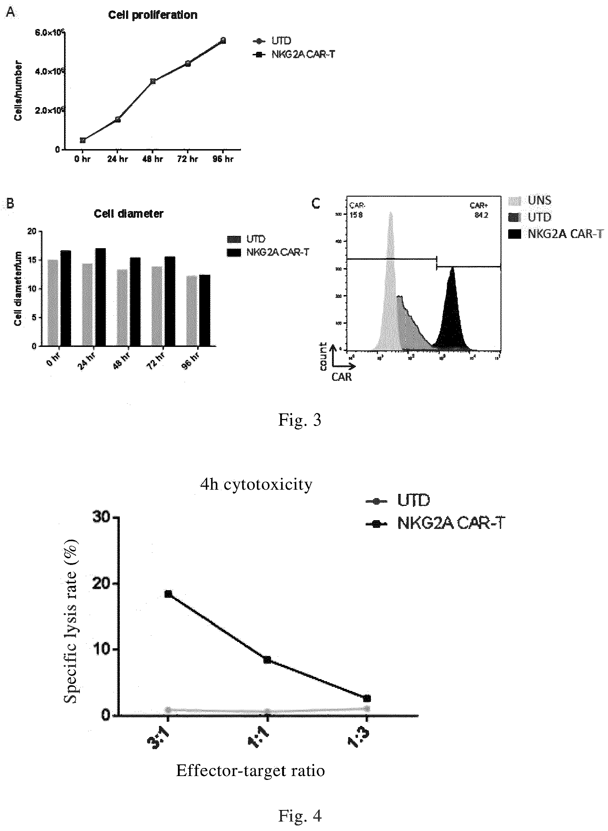

CAR-T cells on day 6 are taken for cell proliferation detection, adjusting the starting cell number to 5*10^5, detecting the cell number at 24hr, 48hr, 72hr, and 96hr, and recording the cell diameter. At the same time, anti-F(ab)'2 antibody is used for flow cytometry staining to detect the expression of CAR vector. The experimental results shows that the NKG2A CAR-T cells exhibit a growth curve similar to that of the untransfected T cells (UTD), and there is no significant difference in the cell diameter of the two kinds of cells. About 80% of the CAR-T cells express CAR molecules targeting NKG2A, indicating that the growth characteristics of NKG2A CAR-T cells are normal (see Fig. 3).

### 2. Preparation of CAR-T Cells Targeting BCMA

Referring to the operation of the above Step 1, a plasmid encoding the chimeric antigen receptor targeting BCMA (amino acid sequence is represented by SEQ ID NO: 6) is constructed. The plasmid map is shown in Fig. 9. The plasmid is packaged by lentivirus, and transfected into T cells to obtain the BCMA-CAR T cells targeting BCMA.

### 3. In Vitro Killing Function Test.

Primary NK cells are amplified in vitro as target cells, adjusting the cell density to 5*10^5/mL, taking 100 µl of the cells to inoculate them into a 96-well plate (making 3 replicate wells in parallel), inoculating the corresponding CAR-T cells in three ratios of effector T cells:target cells (1:3, 1:1, and 3:1). MEM-α+5% FBS is used as the medium to incubate the cells in an incubator at 37°C and 5% CO2 for 4hr and 18hr respectively. Using the cytotox-96 non-radioactive cytotoxicity assay kit (purchased from Thermo Fisher), 50µl of the supernatant is taken for determining the content of lactate dehydrogenase (LDH), calculating the lysis efficiency of primary NK cells in the two groups of UTD and NKG2A CAR-T.

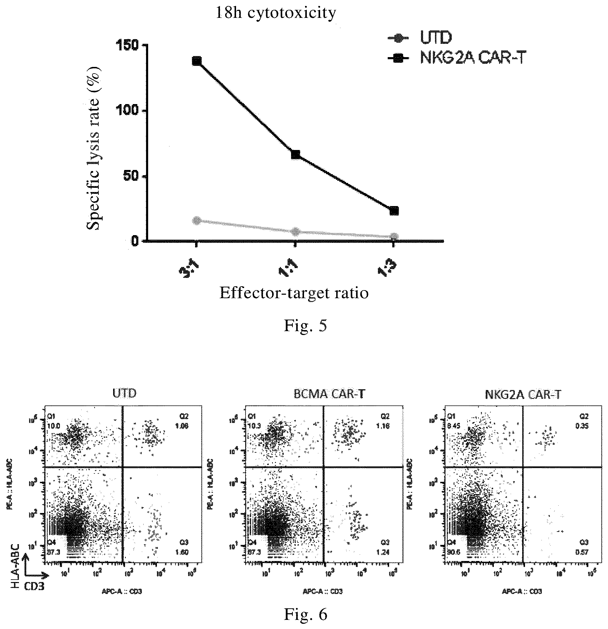

The test results show that the LDH value in the NKG2A CAR-T group is significantly higher than that in the UTD group, indicating that NKG2A CAR-T may effectively kill primary NK cells (see Fig. 4 and Fig. 5).

### Example 3: Preparation of NKG2A UCAR-T Cells

### 1. Knockout of TCR and B2M Genes.

After expanding conventional UTD cells, NKG2A CAR-T cells and BCMA CAR-T cells (as control) in vitro for 48 hours, the cell density is adjusted to 2*10^7/mL. The Cas9 enzyme (purchased from NEB) and sgRNA are incubated at a ratio of 1:4 for 10 minutes at room temperature to obtain the RNP complex solution, wherein the nucleic acid sequence of TRAC-sgRNA is represented by SEQ ID NO: 7, and the nucleic acid sequence of B2M-sgRNA is represented by SEQ ID NO: 8. 1*10^6 cells are mixed with RNP complex solution (the final concentration of Cas9 enzyme is 3µM), using Maxcyte electroporation instruments to separately introduce the RNP complex into CAR-T cells. On day 7 after electroporation, flow cytometry is used to detect the knockout of TCR and B2M genes. The experimental results show that the knockout efficiency of TRAC and B2M is above 85% (see Fig. 6).

### 2. Screening of TCR/B2M Double Negative Cells

Expanding in vitro the CAR-T cells and UTD cells which are knocked out of B2M and TCR, the cell density is adjusted to 1*10^7/mL on day 8, labelling the cells with anti-HLA-ABC and B2M antibodies, then using the secondary antibody conjugated with phycoerythrin (PE) for labeling. After sorting labeled cells with anti-PE magnetic beads through the sorting column, the TCR and B2M double-negative cells are collected (the sorting kit purchased from Miltenyi Biotec). Then, BCMA UCAR-T cells, NKG2A UCAR-T cells and U-UTD cells which lack TCR and B2M are obtained.

### Example 4: Verification of the Resistance Function of NKG2A UCAR-T Cells to NK Cell

### 1. Detecting the rejection of UCAR-T cells against NK cells by LDH test

UTD cells, BCMA-CAR T cells, NKG2A CAR-T cells, BCMA UCAR-T cells, NKG2A UCAR-T cells and U-UTD cells are used as target cells, adjusting the cell concentration to 5*10^5/mL to inoculate 100µl of the cells into a 96-well plate, and inoculating the same volume and number of NK cells in a ratio of primary amplified NK cells to target cells at 1:1, then incubating in an incubator for 4hr and 18hr respectively. 50µl of the supernatant is taken for determining the content of lactate dehydrogenase (LDH), calculating the lysis efficiency of CAR-T and UCAR-T cells. The test results show that the LDH values of the UTD and BCMA CAR-T groups are both very low, indicating that ordinary CAR-T cells will not cause the attack of NK cells, while the U-UTD and BCMA UCAR-T groups exhibit gradually increasing LDH values at 4hr and 18hr, indicating that NK cells will kill T cells lacking TCR and B2M; and NKG2A UCAR-T cells exhibit lower level of LDH, indicating that NKG2A UCAR-T cells are resistant to NK cells.

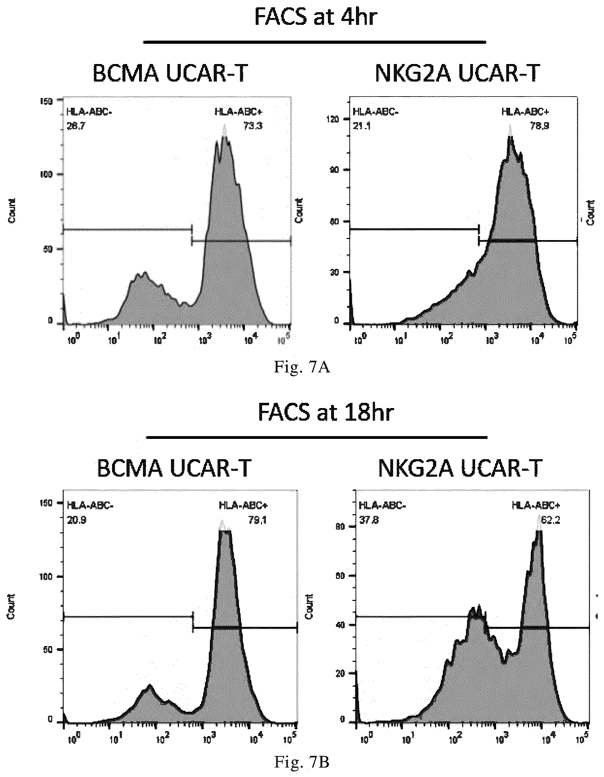

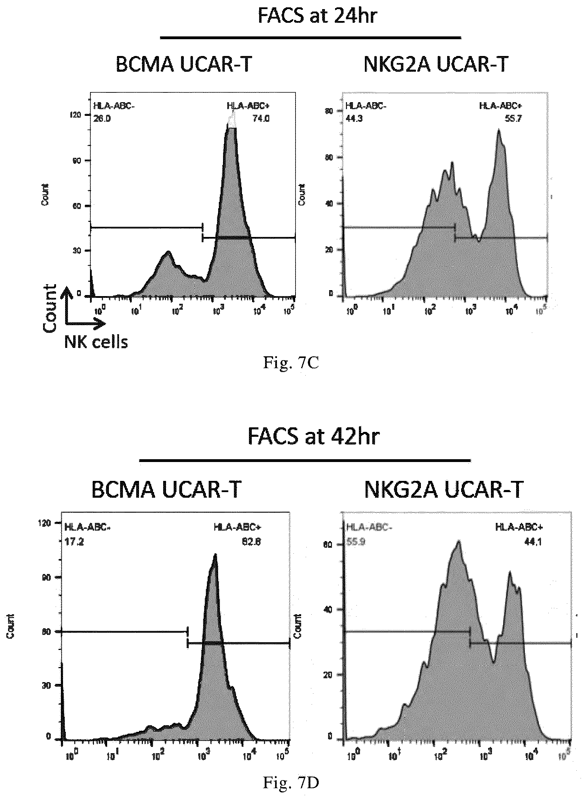

2. In order to further confirm the resistance of NKG2A UCAR-T cells to NK cells, BCMA UCAR-T cells are selected as control, adjusting the cell concentration to 5*10^5/mL to inoculate 100µl of the cells into 96-well plates, and inoculating the same volume and number of NK cells in a ratio of primary expanded NK cells to target cells at 1:1, then incubating in an incubator for 4hr, 18hr, 24hr and 42hr respectively. HLA-ABC positive NK cells are labelled by flow cytometry, detecting the proportion of UCAR-T cells at different time points of co-incubation. The experimental results are shown in Figs. 7A-7D. BCMA UCAR-T is at a low ratio of about 20% at 4 hours, and the ratio at a very low level is kept with the extension of the detection time, indicating that NK cells significantly inhibit the growth of BCMA UCAR-T cells; while NKG2A UCAR-T cells are at a low ratio of about 20% at 4hr, with the extension of the detection time, they exhibit a gradually increasing ratio, reaching nearly 60% at 42hr, indicating that the growth of NKG2A UCAR-T cells is initially inhibited by NK cells, but the NKG2A UCAR-T cells gradually restore their proliferation ability over time. The above results indicate that NKG2A UCAR-T cells may effectively resist the killing of NK cells.

3. In order to further prove the resistance of NKG2A UCAR-T cells to primary NK cells, BCMA UCAR-T cells and NKG2A UCAR-T cells expressing GFP are constructed. The amino acid sequence of BCMA-GFP is represented by SEQ ID NO: 24, and the amino acid sequence of NKG2A-GFP is represented by SEQ ID NO: 25.

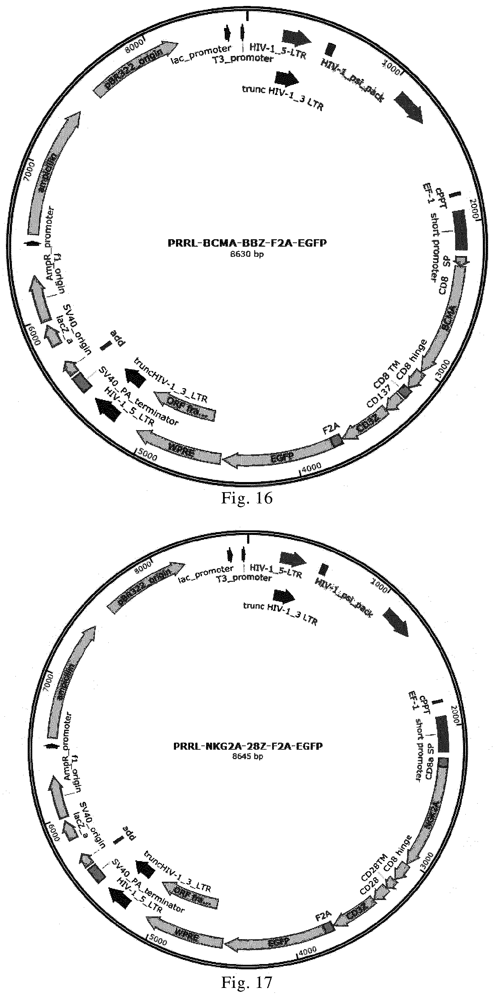

Referring to the operation in Example 2, the plasmid PRRL-BCMA-BBZ-F2A-EGFP expressing GFP is constructed for BCMA UCAR-T cells. The plasmid map is shown in Fig. 16. The plasmid PRRL-NKG2A-28Z-F2A-EGFP expressing GFP is constructed for NKG2A UCAR-T cells, the plasmid map is shown in Fig. 17. The constructed plasmid is packaged by lentivirus, and transfected into T cells, performing gene knockout and magnetic bead sorting for CAR-T cells to obtain BCMA UCAR-T cells expressing GFP and NKG2A UCAR-T cells expressing GFP.

The concentration of CAR-T cells is adjusted to 5*10^5/mL to inoculate 100µl of the cells into 96-well plate, inoculating the same volume and number of NK cells in a ratio of primary expanded NK cells to target cells at 1:1, then incubating in an incubator for 0hr, 4hr, 18hr, 24hr and 48hr respectively. Flow cytometry is used to detect the proportion of GFP cells at different time points of co-incubation, so as to track the survival of UCAR-T cells.

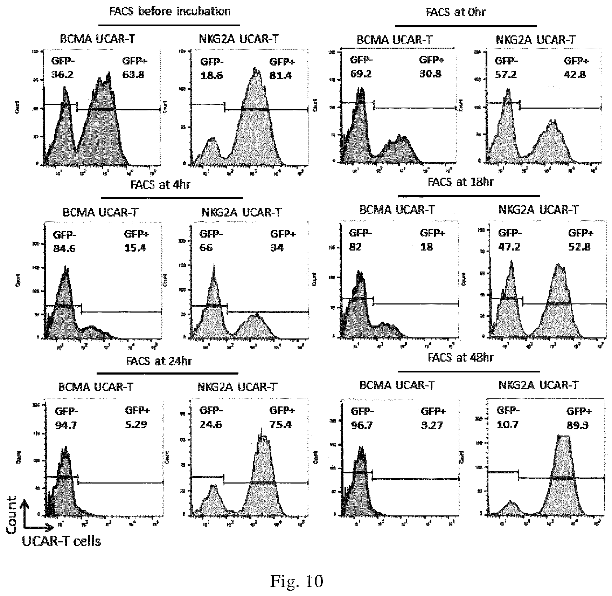

The results of the experiment are shown in Fig. 10. The proportion of GFP-positive BCMA UCAR-T cells is gradually decreasing over time, after 48 hours they are basically completely killed by NK cells; while the proportion of GFP-positive NKG2A UCAR-T cells decreases slightly within 4 hours, increases significantly after 18 hours, and accounts for about 90% after 48 hours, indicating that NKG2A UCAR-T cells may significantly resist the killing of NK cells.

### Example 5: Resistance of NKG2A UCAR-T Cells to NK Cells in Vivo

BCMA UCAR-T and NKG2A UCAR-T cells are cultured in vitro, adjusting the CAR positive rate to 80%, injecting the cells into NPG immunodeficient mice through the tail vein at a dose of 8*10^6 cells/mouse, and dividing the mice into two groups: BCMA UCAR-T and NK cell group (labeled as BCMA UCAR-T+NK), and NKG2A UCAR-T and NK cell group (labeled as NKG2A-UCART+NK).

4hr after administering UCAR T cells, the same amount of NK cells are injected. On day 1, 3, and 6 after injecting the CAR T cells, the survival of human-derived CD4 and CD8 T cells in the peripheral blood of the mice are detected by flow absolute technology.

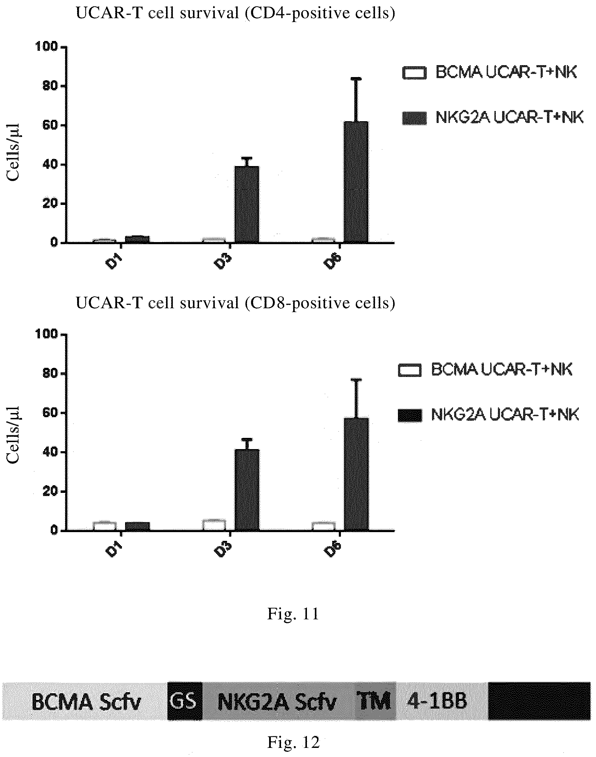

The results of the experiment are shown in Fig. 11. On day 1 after injection, the number of UCAR-T cells (i.e., human-derived CD4 and CD8 T cells) in the BCMA UCAR-T+NK group and NKG2A UCAR-T+NK group decreases significantly, indicating that UCAR-T cells are rejected by NK cells. On day 3 and 6 after injection, the number of UCAR-T cells in the BCMA UCAR-T+NK group is very low, while the number of UCAR-T cells in the NKG2A UCAR-T+NK group increases significantly on day 3 and 6. The above results show that in the in vivo model, NK cells significantly inhibit the survival of BCMA UCAR-T cells, and NKG2A UCAR-T cells may effectively resist the killing of NK cells, and restore their proliferation ability.

### Example 6: Construction of CAR T Cells Targeting BCMA and NKG2A

As shown in Fig. 12, UCAR-T cells (i.e., BCMA-GS-NKG2A UCAR-T) with scFv targeting BCMA and scFv targeting NKG2A in tandem are prepared. The amino acid sequence of BCMA-GS-NKG2A CAR is represented by SEQ ID NO: 9.

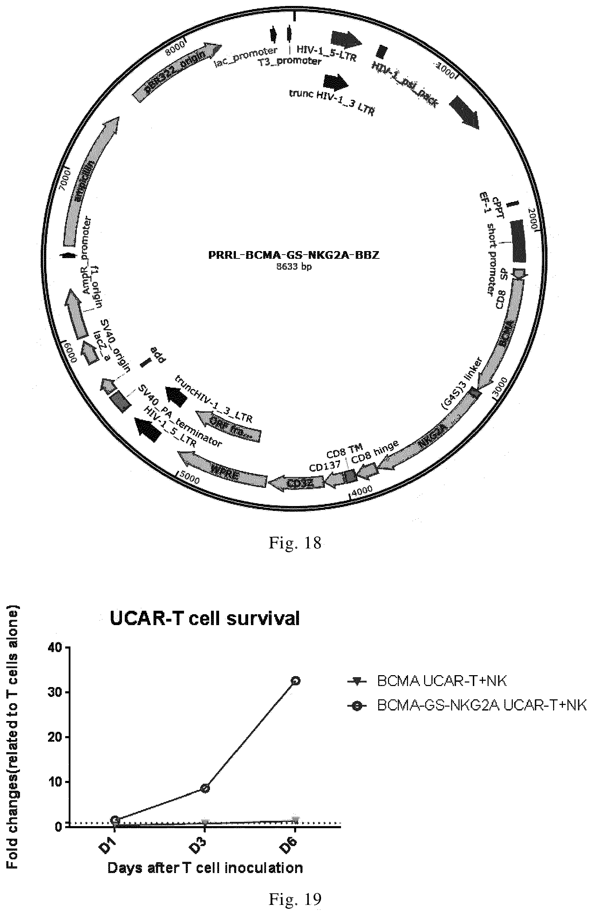

The plasmid PRRL-BCMA-GS-NKG2A-BBZ for BCMA-GS-NKG2A UCAR-T is constructed, and the plasmid map is shown in Fig. 18. Referring to the operations in Examples 2 and 3, virus transfection is performed to obtain BCMA-GS-NKG2A UCAR-T cells, and BCMA-GS-NKG2A UCAR-T cells are subjected to knocking out the TRAC and B2M genes, then more than 99% TCR and HLA-ABC negative BCMA-GS-NKG2A UCAR-T cells are obtained by magnetic bead sorting method.

Referring to the operations in Examples 2 and 3, BCMA UCAR-T cells and NKG2A UCAR-T cells are prepared respectively.

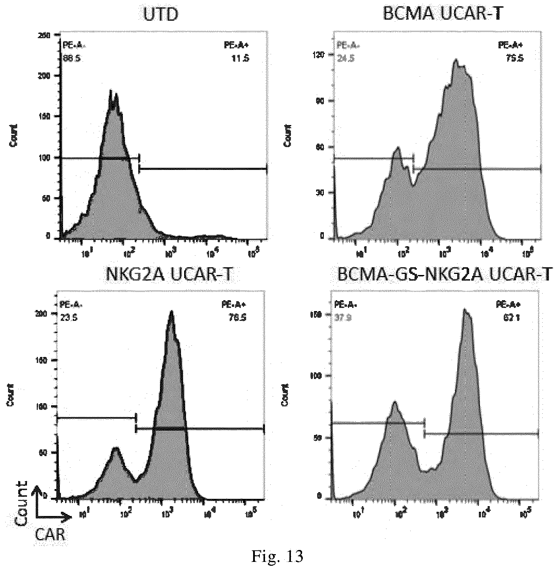

CAR expression of BCMA UCAR-T, NKG2A UCAR-T and BCMA-GS-NKG2A UCAR-T are detected respectively. The experimental results are shown in Fig. 13. The positive rate is above 60%, indicating that the BCMA-GS-NKG2A UCAR-T cells are successfully prepared.

### Example 7: In Vitro Functional Verification of BCMA-GS-NKG2A UCAR-T Cells

BCMA-positive multiple myeloma cell lines RPMI-8226 and NCI-H929 are cultured in vitro as target cells, 1*10^4 tumor cells are inoculated on 96-well plates, and the corresponding number of UCAR-T cells are inoculated in a ratio of T cells to tumor cells respectively at 3:1, 1:1 and 1:3, after incubating for 18 hours, 50µl of supernatant is aspirated for detecting the content of LDH.

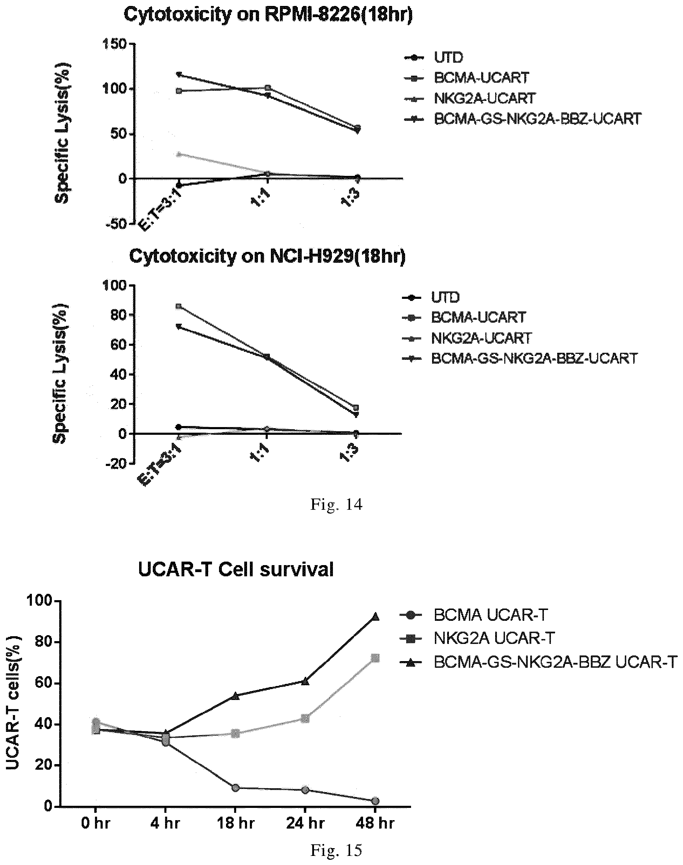

The experimental results are shown in Fig. 14. In the UTD and NKG2A UCAR-T groups, the cell lysis rate of RPMI-8226 and NCI-H929 is very low; while the tumor cell lysis rate of the BCMA-GS-NKG2A UCAR-T group is equivalent to that of BCMA UCAR-T group, indicating that BCMA-GS-NKG2A UCAR-T cells may effectively kill BCMA-positive tumor cells in vitro.

### Example 8: Verification of the Resistance Function of BCMA-GS-NKG2A UCAR-T Cells to NK Cells

BCMA UCAR-T and NKG2A UCAR-T cells are selected as negative and positive controls respectively, adjusting the cell concentration to 5*10^5/mL to inoculate 100µl of the cells to 96-well plate, inoculating the same volume and number of NK cells in a ratio of NK cells to T cells at 1:1 to incubate in an incubator for 0hr, 4hr, 18hr, 24hr and 48hr respectively. Flow cytometry is used to label HLA-ABC positive NK cells, detecting the proportion of UCAR-T cells at different time points of co-incubation. The experimental results are shown in Fig. 15. As the incubation time increases, the proportion of BCMA UCAR-T cells gradually decreases, and they are basically killed by NK cells in 48hr; while BCMA-GS-NKG2A UCAR-T and NKG2A UCAR-T cells show the same trend of change, a slight decrease is found at 4hr, and then the cells gradually increase, reaching more than 70% at 48hr, and the proportion of BCMA-GS-NKG2A UCAR-T cells reaches 90% at 48hr. The above results indicate that BCMA-GS-NKG2A UCAR-T cells may effectively resist the killing of NK cells.

### Example 9: Resistance of BCMA-GS-NKG2A UCAR-T Cells to NK cells in Vivo

BCMA UCAR-T and BCMA-GS-NKG2A UCAR-T cells are cultured in vitro, adjusting the CAR positive rate to 60%, and injecting the cells into NPG immunodeficient mice through the tail vein at a dose of 8*10^6 cells/mouse. The mice are divided into two groups: the BCMA UCAR-T and NK cell group (labeled as BCMA UCAR-T+NK), and the BCMA-GS-NKG2A UCAR-T and NK cell group (labeled as BCMA-GS-NKG2A UCAR-T+NK). 4 hours after administering UCAR-T cells, the same amount of NK cells is injected. On day 1, 3 and 6 after the CAR T cell injection, the survival of human-derived CD45-positive T cells in the peripheral blood of the mice is detected by flow absolute technology.

The experimental results are shown in Fig. 19. Compared with day 1 after injection, on day 3 and 6 the number of UCAR-T cells in the BCMA UCAR-T+NK group doesn't increase significantly, indicating that UCAR-T cells are rejected by NK cells. However, on day 3 and 6 the number of UCAR-T cells in the BCMA-GS-NKG2A UCAR-T+NK group increases significantly, and the number of the cells on day 6 increases by more than 30 times compared with that on day 1. The above results indicate that in the in vivo model, NK cells significantly inhibit the survival of BCMA UCAR-T cells, and BCMA-GS-NKG2A UCAR-T cells may effectively resist the killing of NK cells, and restore their proliferation ability.

The sequences involved in the present application are shown in the following table:

| SEQ ID NO: | NAMES | SEQUENCES |
|---|---|---|
| 1 | Anti-NKG2A antibody-VH | QVQLVQSGAEVKKPGASVKVSCKASGYTFTSYWMNWVRQAPGQGLEWMGRIDPY DSETHYAQKLQGRVTMTTDTSTSTAYMELRSLRSDDTAVYYCARGGYDFDVGTLYW FFDVWGQGTTVTVSS |
| 2 | Anti-NKG2A antibody-VL | DIQMTQSPSSLSASVGDRVTITCRASENIYSYLAWYQQKPGKAPKLLIYNAKTLAEGVP SRFSGSGSGTDFTLTISSLQPEDFATYYCQHHYGTPRTFGGGTKVEIK |
| 3 | Transmembrane and intracellular domains of CD28 | FwvlvvvggvlacysllvtvatiifwvRskrsrllhsdymnmtprrpgptrkhyqpyapprdfaayrs |
| 4 | T cell activating factor CD3ζ | Rvkfsrsadapayqqgqnqlynelnlgrreeydvldkrrgrdpemggkpqrrknpqeglynelqkdkma eayseigmkgerrrgkghdglyqglstatkdtydalhmqalppr |
| 5 | NKG2A CAR | MALPVTALLLPLALLLHAARPQVQLVQSGAEVKKPGASVKVSCKASGYTFTSYWMN WVRQAPGQGLEWMGRIDPYDSETHYAQKLQGRVTMTTDTSTSTAYMELRSLRSDD TAVYYCARGGYDFDVGTLYWFFDVWGQGTTVTVSSGGGGSGGGGSGGGGSDIQM TQSPSSLSASVGDRVTITCRASENIYSYLAWYQQKPGKAPKLLIYNAKTLAEGVPSRFS GSGSGTDFTLTISSLQPEDFATYYCQHHYGTPRTFGGGTKVEIKTTTPAPRPPTPAPTI ASQPLSLRPEACRPAAGGAVHTRGLDFACDFWVLVVVGGVLACYSLLVTVAFIIFWV RSKRSRLLHSDYMNMTPRRPGPTRKHYQPYAPPRDFAAYRSRVKFSRSADAPAYQQ GQNQLYNELNLGRREEYDVLDKRRGRDPEMGGKPQRRKNPQEGLYNELQKDKMA EAYSEIGMKGERRRGKGHDGLYQGLSTATKDTYDALHMQALPPR |
| 6 | BCMA-CAR | MALPVTALLLPLALLLHAARPEVQLLESGGGLVQPGGSLRLSCAASGFTFGGNAMS WVRQAPGKGLEWVSAISGNGGSTFYADSVKGRFTISRDNSKNTLYLQMNSLRAED TAVYYCAKVRPFWGTFDYWGQGTLVTVSSGGGGSGGGGSGGGGSEIVLTQSPGT LSLSPGERATLSCRASQSVSSSYLAWYQQKPGQAPRLLIYGASSRATGIPDRFSGSG SGTDFTLTISRLEPEDFAVYYCQQYFNPPEYTFGQGTKVEIKRTTTPAPRPPTPAPTIA SQPLSLRPEACRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAPAYQQGQNQ LYNELNLGRREEYDVLDKRRGRDPEMGGKPQRRKNPQEGLYNELQKDKMAEAYSE IGMKGERRRGKGHDGLYQGLSTATKDTYDALHMQALPPR |
| 7 | TRAC-sgRNA | AGAGTCTCTCAGCTGGTACA |
| 8 | B2M-sgRNA | GAGTAGCGCGAGCACAGCTA |
| 9 | BCMA-GS-NKG2A CAR | MALPVTALLLPLALLLHAARPEVQLLESGGGLVQPGGSLRLSCAASGFTFGGNAMSW VRQAPGKGLEWVSAISGNGGSTFYADSVKGRFTISRDNSKNTLYLQMNSLRAEDTAV YYCAKVRPFWGTFDYWGQGTLVTVSSGGGGSGGGGSGGGGSEIVLTQSPGTLSLSP GERATLSCRASQSVSSSYLAWYQQKPGQAPRLLIYGASSRATGIPDRFSGSGSGTDFT LTISRLEPEDFAVYYCQQYFNPPEYTFGQGTKVEIKRGGGGSGGGGSGGGGSQVQLV QSGAEVKKPGASVKVSCKASGYTFTSYWMNWVRQAPGQGLEWMGRIDPYDSETHY |
| | | AQKLQGRVTMTTDTSTSTAYMELRSLRSDDTAVYYCARGGYDFDVGTLYWFFDVWG QGTTVTVSSGGGGSGGGGSGGGGSDIQMTQSPSSLSASVGDRVTITCRASENIYSYLA WYQQKPGKAPKLLIYNAKTLAEGVPSRFSGSGSGTDFTLTISSLQPEDFATYYCQHHYG TPRTFGGGTKVEIKTTTPAPRPPTPAPTIASQPLSLRPEACRPAAGGAVHTRGLDFACDI YIWAPLAGTCGVLLLSLVITLYCKRGRKKLLYIFKQPFMRPVQTTQEEDGCSCRFPEEEEG GCELRVKFSRSADAPAYQQGQNQLYNELNLGRREEYDVLDKRRGRDPEMGGKPQRR KNPQEGLYNELQKDKMAEAYSEIGMKGERRRGKGHDGLYQGLSTATKDTYDALHMQ ALPPR |
| 10 | Anti-NKG2A antibody-HCDR1 | SYWMN |
| 11 | Anti-NKG2A antibody-HCDR2 | RIDPYDSETHYAQKLQG |
| 12 | Anti-NKG2A antibody-HCDR3 | GGYDFDVGTLYWFFDV |
| 13 | Anti-NKG2A antibody-LCDR1 | RASENIYSYLA |
| 14 | Anti-NKG2A antibody-LCDR2 | NAKTLAE |
| 15 | Anti-NKG2A antibody-LCDR3 | QHHYGTPRT |
| 16 | Anti-BCMA antibody-HCDR1 | GNAMS |
| 17 | Anti-BCMA antibody-HCDR2 | AISGNGGSTFYADSVKG |
| 18 | Anti-BCMA antibody-HCDR3 | VRPFWGTFDY |
| 19 | Anti-BCMA antibody-LCDR1 | RASQSVSSSYLA |
| 20 | Anti-BCMA antibody-LCDR2 | GASSRAT |
| 21 | Anti-BCMA antibody-LCDR3 | QQYFNPPEYT |
| 22 | Anti-BCMA antibody-VH | EVQLLESGGGLVQPGGSLRLSCAASGFTFGGNAMSWVRQAPGKGLEWVSAISGNGGS TFYADSVKGRFTISRDNSKNTLYLQMNSLRAEDTAVYYCAKVRPFWGTFDYWGQGTLV TVSS |
| 23 | Anti-BCMA antibody-VL | EIVLTQSPGTLSLSPGERATLSCRASQSVSSSYLAWYQQKPGQAPRLLIYGASSRATGIPD RFSGSGSGTDFTLTISRLEPEDFAVYYCQQYFNPPEYTFGQGTKVEIKR |
| 24 | BCMA-GFP amino acid sequence | EVQLLESGGGLVQPGGSLRLSCAASGFTFGGNAMSWVRQAPGKGLEWVSAISGNGGS TFYADSVKGRFTISRDNSKNTLYLQMNSLRAEDTAVYYCAKVRPFWGTFDYWGQGTLV TVSS EIVLTQSPGTLSLSPGERATLSCRASQSVSSSYLAWYQQKPGQAPRLLIYGASSRATGIPD RFSGSGSGTDFTLTISRLEPEDFAVYYCQQYFNPPEYTFGQGTKVEIKR MALPVTALLLPLALLLHAARPEVQLLESGGGLVQPGGSLRLSCAASGFTFGGNAMSW VRQAPGKGLEWVSAISGNGGSTFYADSVKGRFTISRDNSKNTLYLQMNSLRAEDTAV YYCAKVRPFWGTFDYWGQGTLVTVSSGGGGSGGGGSGGGGSEIVLTQSPGTLSLSP GERATLSCRASQSVSSSYLAWYQQKPGQAPRLLIYGASSRATGIPDRFSGSGSGTDFT LTISRLEPEDFAVYYCQQYFNPPEYTFGQGTKVEIKRTTTPAPRPPTPAPTIASQPLSLRP EACRPAAGGAVHTRGLDFACDIYIWAPLAGTCGVLLLSLVITLYCKRGRKKLLYIFKQPF MRPVQTTQEEDGCSCRFPEEEEGGCELRVKFSRSADAPAYQQGQNQLYNELNLGRR |
| | | EEYDVLDKRRGRDPEMGGKPQRRKNPQEGLYNELQKDKMAEAYSEIGMKGERRRGK GHDGLYQGLSTATKDTYDALHMQALPPRVKQTLNFDLLKLAGDVESNPGPMVSKGE ELFTGVVPILVELDGDVNGHKFSVSGEGEGDATYGKLTLKFICTTGKLPVPWPTLVTTLT YGVQCFSRYPDHMKQHDFFKSAMPEGYVQERTIFFKDDGNYKTRAEVKFEGDTLVNR IELKGIDFKEDGNILGHKLEYNYNSHNVYIMADKQKNGIKVNFKIRHNIEDGSVQLADH YQQNTPIGDGPVLLPDNHYLSTQSALSKDPNEKRDHMVLLEFVTAAGITLGMDELYK |
| 25 | NKG2A-GFP amino acid sequence | MALPVTALLLPLALLLHAARPQVQLVQSGAEVKKPGASVKVSCKASGYTFTSYWMN WVRQAPGQGLEWMGRIDPYDSETHYAQKLQGRVTMTTDTSTSTAYMELRSLRSDD TAVYYCARGGYDFDVGTLYWFFDVWGQGTTVTVSSGGGGSGGGGSGGGGSDIQM TQSPSSLSASVGDRVTITCRASENIYSYLAWYQQKPGKAPKLLIYNAKTLAEGVPSRFS GSGSGTDFTLTISSLQPEDFATYYCQHHYGTPRTFGGGTKVEIKTTTPAPRPPTPAPTIA SQPLSLRPEACRPAAGGAVHTRGLDFACDFWVLVVVGGVLACYSLLVTVAFIIFWVRS KRSRLLHSDYMNMTPRRPGPTRKHYQPYAPPRDFAAYRSRVKFSRSADAPAYQQGQ NQLYNELNLGRREEYDVLDKRRGRDPEMGGKPQRRKNPQEGLYNELQKDKMAEAY SEIGMKGERRRGKGHDGLYQGLSTATKDTYDALHMQALPPRVKQTLNFDLLKLAGD VESNPGPMVSKGEELFTGVVPILVELDGDVNGHKFSVSGEGEGDATYGKLTLKFICTT GKLPVPWPTLVTTLTYGVQCFSRYPDHMKQHDFFKSAMPEGYVQERTIFFKDDGNY KTRAEVKFEGDTLVNRIELKGIDFKEDGNILGHKLEYNYNSHNVYIMADKQKNGIKVN FKIRHNIEDGSVQLADHYQQNTPIGDGPVLLPDNHYLSTQSALSKDPNEKRDHMVLL EFVTAAGITLGMDELYK |

## Claims

1. A T cell resistant to transplantation immune rejection,
wherein the T cell expresses a first protein that is a chimeric receptor recognizing NKG2A on NK cells of a host;
wherein the chimeric receptor comprises an extracellular domain having an antigen binding region recognizing NKG2A, a transmembrane domain, and an intracellular signaling domain; and
wherein in the T cell the HLA-I gene and the endogenous TCR gene are silenced.

2. The T cell according to claim 1, wherein the chimeric receptor is a chimeric antigen receptor (CAR), a chimeric T cell receptor, or a T cell antigen coupler (TAC).

3. The T cell according to claim 1 or 2, wherein the antigen binding region is selected from the group consisting of scFv, Fv, Fab, Fab', Fab'-SH, F(ab')₂, single domain fragment, and natural ligand engaging its homologous receptor.

4. The T cell according to any one of claims 1 to 3, wherein the first protein comprises an antigen binding region comprising HCDR1 sequence of SEQ ID NO: 10, HCDR2 sequence of SEQ ID NO: 11, HCDR3 sequence of SEQ ID NO: 12; and LCDR1 sequence of SEQ ID NO: 13, LCDR2 sequence of SEQ ID NO: 14, LCDR3 sequence of SEQ ID NO: 15.

5. The T cell according to claim 4, wherein the first protein comprises an antigen binding region comprising a heavy chain variable region sequence of SEQ ID NO: 1, or a light chain variable region sequence of SEQ ID NO: 2.

6. The T cell according to claim 4 or 5, wherein the amino acid sequence of the first protein is represented by SEQ ID NO: 5.

7. The T cell according to any one of claims 1 to 6, wherein the HLA-I gene is one or more genes selected from the group consisting of: HLA-A, HLA-B, HLA-C, and B2M.

8. The T cell according to claim 7, wherein the HLA-I gene is B2M.

9. The T cell according to any one of claims 1 to 8, wherein the HLA-I gene and the endogenous TCR gene are silenced by gene editing technology.

10. The T cell according to claim 9, wherein the gene editing technology is selected from the group consisting of: CRISPR/Cas9 technology, artificial zinc finger nuclease (ZFN) technology, transcription activator-like effector (TALE) technology, and TALE-CRISPR/Cas9 technology.

11. The T cell according to claim 10, wherein the gene editing technology is CRISPR/Cas9 technology.

12. The T cell according to any one of claims 1 to 11, wherein the first protein further comprises an antigen binding region recognizing a tumor antigen or a pathogen antigen.

13. The T cell according to claim 12, wherein the antigen binding region recognizing NKG2A of the host and the antigen binding region recognizing a tumor antigen or a pathogen antigen are connected by a linker peptide.

14. The T cell according to claim 12 or 13, wherein the first protein comprises the amino acid sequence of SEQ ID NO: 9.

15. The T cell according to any one of claims 1 to 11, wherein the T cell further expresses a second protein recognizing a tumor antigen or a pathogen antigen.

16. The T cell according to claim 15, wherein the second protein is a chimeric receptor.

17. The T cell according to claim 16, wherein the chimeric receptor is a CAR, a chimeric T cell receptor, or a TAC.

18. The T cell according to any one of claims 15 to 17, wherein the second protein comprises an extracellular domain, a transmembrane domain, and an intracellular signaling domain.

19. The T cell according to any one of claims 15 to 18, wherein the second protein comprises an antigen binding region specifically recognizing BCMA.

20. The T cell according to claim 19, wherein the antigen binding region specifically recognizing BCMA comprises HCDR1 sequence of SEQ ID NO: 16, HCDR2 sequence of SEQ ID NO: 17, HCDR3 sequence of SEQ ID NO: 18, and LCDR1 sequence of SEQ ID NO: 19, LCDR2 sequence of SEQ ID NO: 20, LCDR3 sequence of SEQ ID NO: 21.

21. The T cell according to claim 19, wherein the antigen binding region specifically recognizing BCMA comprises a heavy chain variable region sequence of SEQ ID NO: 22 and a light chain variable region sequence of SEQ ID NO: 23.

22. The T cell according to claim 19, wherein the amino acid sequence of the second protein is represented by SEQ ID NO:6.

23. The T cell according to any one of claims 12 to 18, wherein the tumor antigen comprises thyroid stimulating hormone receptor (TSHR); CD171; CS-1; C-type lectin-like molecule-1; ganglioside GD3; Tn antigen; CD19; CD20; CD 22; CD 30; CD70; CD123; CD138; CD33; CD44; CD44v7/8; CD38; CD44v6; B7H3(CD276), B7H6; KIT(CD 117); interleukin 13 receptor subunit a (IL-13Ra); interleukin 11 receptor a (IL-1 lRa); prostate stem cell antigen (PSCA); prostate specific membrane antigen (PSMA); carcinoembryonic antigen (CEA); NY-ESO-1; HIV-lGag; MART-1; gp100; tyrosinase; mesothelin; EpCAM; protease serine 21 (PRSS21); vascular endothelial growth factor receptor, vascular endothelial growth factor receptor 2 (VEGFR2); Lewis (Y) antigen; CD24; platelet derived growth factor receptor β (PDGFR-β); stage-specific embryonic antigen-4 (SSEA-4); cell surface-associated mucin 1 (MUCl), MUC6; epidermal growth factor receptor family and its mutants (EGFR, EGFR2, ERBB3, ERBB4, EGFRvIII); neural cell adhesion molecule (NCAM); carbonic anhydrase IX (CAIX); LMP2; ephrin A receptor 2 (EphA2); fucosyl GMl; sialyl Lewis adhesion molecule (sLe); ganglioside GM3; TGS5; high molecular weight melanoma-associated antigen (HMWMAA); O-acetyl GD2 ganglioside (OAcGD2); folic acid receptor; tumor vascular endothelial marker 1 (TEM1/CD248); tumor vascular endothelial marker 7 related (TEM7R); Claudin 6, Claudin 18.2, Claudin 18.1; ASGPRI; CDH16; 5T4; 8H9; αvβ6 integrin; B cell maturation antigen (BCMA); CA9; 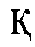 light chain (kappa light chain); CSPG4; EGP2, EGP40; FAP; FAR; FBP; embryonic AchR; HLA-Al, HLA-A2; MAGEAl, MAGE3; KDR; MCSP; NKG2D ligand; PSC1; ROR1; Sp17; SURVIVIN; TAG72; TEMl; conectin; tenascin; carcinoembryonic variant of tumor necrosis zone; G protein-coupled receptor C group 5-member D (GPRC5D); X chromosome open reading frame 61 (CXORF61); CD97; CD179a; anaplastic lymphoma kinase (ALK); polysialic acid; placenta-specific 1 (PLACl); hexose moiety of globoH glycoceramide (GloboH); breast differentiation antigen (NY-BR-1); uroplakin 2 (UPK2); hepatitis A virus cell receptor 1 (HAVCRl); adrenergic receptor β3 (ADRB3); pannexin 3 (PANX3); G protein-coupled receptor 20 (GPR20); lymphocyte antigen 6 complex locus K9 (LY6K); olfactory receptor 51E2 (OR51E2); TCRy alternating reading frame protein (TARP); Wilms tumor protein (WTl); ETS translocation variant gene 6 (ETV6-AML); sperm protein 17 (SPA17); X antigen family member lA (XAGE1); angiogenin binding cell surface receptor 2 (Tie2); melanoma cancer-testis antigen-1 (MAD-CT-1); melanoma cancer-testis antigen-2 (MAD-CT-2); Fas-related antigen 1; p53 mutant; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosaminyl transferase V (NA17); paired box protein Pax-3 (PAX3); androgen receptor; cyclin Bl; V-myc avian myeloidosis virus oncogene neuroblastoma-derived homolog (MYCN); Ras homolog family member C (RhoC); cytochrome P450 lBl (CYPlBl); CCCTC binding factor (zinc finger protein)-like (BORIS); squamous cell carcinoma antigen 3 (SART3) recognized by T cells; paired box protein Pax-5 (PAX5); proacrosin binding protein sp32 (OYTES1); lymphocyte-specific protein tyrosine kinase (LCK); A kinase anchoring protein 4 (AKAP-4 ); synovial sarcoma X breakpoint 2 (SSX2); CD79a; CD79b; CD72; leukocyte-associated immunoglobulin-like receptor 1 (LAIR1); Fe fragment of IgA receptor (FCAR); leukocyte immunoglobulin-like receptor subfamily member 2 (LILRA2); CD300 molecular-like family member f (CD300LF); C-type lectin domain family 12 member A (CLEC12A); bone marrow stromal cell antigen 2 (BST2); containing EGF-like module mucin-like hormone receptor like 2 (EMR2); lymphocyte antigen 75 (LY75); glypican-3 (GPC3); Fc receptor-like 5 (FCRL5); immunoglobulin lambda-like polypeptide 1 (IGLL1).

24. The T cell according to any one of claims 12 to 18, wherein the pathogen antigen is selected from a group consisting of a virus antigen, a bacteria antigen, a fungus antigen, a protozoa antigen, and a parasite antigen, optionally wherein the virus antigen is selected from a group consisting of a cytomegalovirus antigen, an Epstein-Barr virus antigen, a human immunodeficiency virus antigen, and an influenza virus antigen.

25. The T cell according to any one of claims 9 to 24, wherein the HLA-1 gene is silenced using a sgRNA, the sequence of which is represented by SEQ ID NO: 8, and/or the TCR gene is silenced using a sgRNA, the sequence of which is represented by SEQ ID NO: 7.

26. The T cell according to any one of claims 1 to 25, wherein the T cell is an autologous T cell or an allogeneic T cell.

27. A T cell as defined according to any one of claims 1 to 26 for use in a method for preventing or regulating transplantation immune rejection.

28. A T cell as defined according to any one of claims 1 to 26 for use in a method for preventing or regulating the attack of NK cell on exogenous cells.

29. The T cell for use of claim 28, wherein the exogenous cells are (i) T cells, NK T cells, or stem cells; or (ii) engineered T cells, NK T cells, or stem cells.

## Patentansprüche

1. T-Zelle, die gegenüber immunologischer Transplantationsabstoßung resistent ist, wobei die T-Zelle ein erstes Protein exprimiert, das ein chimärer Rezeptor ist, der NKG2A auf NK-Zellen eines Wirts erkennt;
wobei der chimäre Rezeptor eine extrazelluläre Domäne, die eine Antigenbindungsregion aufweist, die NKG2A erkennt, eine Transmembrandomäne und eine intrazelluläre Signalisierungsdomäne umfasst; und
wobei in der T-Zelle das HLA-I-Gen und das endogene TCR-Gen stillgelegt sind.

2. T-Zelle nach Anspruch 1, wobei der chimäre Rezeptor ein chimärer Antigenrezeptor (CAR), ein chimärer T-Zell-Rezeptor oder ein T-Zell-Antigen-Koppler (TAC) ist.

3. T-Zelle nach Anspruch 1 oder 2, wobei die Antigenbindungsregion ausgewählt ist aus der Gruppe, bestehend aus scFv, Fv, Fab, Fab', Fab'-SH, F(ab')₂, Einzeldomänenfragment und einem natürlichen Liganden, der an seinen homologen Rezeptor bindet.

4. T-Zelle nach einem der Ansprüche 1 bis 3, wobei das erste Protein eine Antigenbindungsregion umfasst, die die HCDR1-Sequenz von SEQ ID NR: 10, die HCDR2-Sequenz von SEQ ID NR: 11, die HCDR3-Sequenz von SEQ ID NR: 12; und die LCDR1-Sequenz von SEQ ID NR: 13, die LCDR2-Sequenz von SEQ ID NR: 14 und die LCDR3-Sequenz von SEQ ID NR: 15 umfasst.

5. T-Zelle nach Anspruch 4, wobei das erste Protein eine Antigenbindungsregion umfasst, die eine Sequenz der variablen Region der schweren Kette von SEQ ID NR: 1 oder eine Sequenz der variablen Region der leichten Kette von SEQ ID NR: 2 umfasst.

6. T-Zelle nach Anspruch 4 oder 5, wobei die Aminosäuresequenz des ersten Proteins durch SEQ ID NR: 5 dargestellt ist.

7. T-Zelle nach einem der Ansprüche 1 bis 6, wobei das HLA-I-Gen ein oder mehrere Gene ist, die ausgewählt sind aus der Gruppe, bestehend aus: HLA-A, HLA-B, HLA-C und B2M.

8. T-Zelle nach Anspruch 7, wobei das HLA-I-Gen B2M ist.

9. T-Zelle nach einem der Ansprüche 1 bis 8, wobei das HLA-I-Gen und das endogene TCR-Gen durch Geneditierungstechnologie stillgelegt sind.

10. T-Zelle nach Anspruch 9, wobei die Geneditierungstechnologie ausgewählt ist aus der Gruppe, bestehend aus CRISPR/Cas9-Technologie, künstlicher Zinkfingernuklease-(ZFN)-Technologie, transkriptionsaktivatorartiger-Effektor-(TALE)-Technologie und TALE-CRISPR/Cas9-Technologie.

11. T-Zelle nach Anspruch 10, wobei die Geneditierungstechnologie die CRISPR/Cas9-Technologie ist.

12. T-Zelle nach einem der Ansprüche 1 bis 11, wobei das erste Protein weiter eine Antigenbindungsregion umfasst, die ein Tumorantigen oder ein Pathogenantigen erkennt.

13. T-Zelle nach Anspruch 12, wobei die Antigenbindungsregion, die NKG2A des Wirts erkennt, und die Antigenbindungsregion, die ein Tumorantigen oder ein Pathogenantigen erkennt, durch ein Linker-Peptid verbunden sind.

14. T-Zelle nach Anspruch 12 oder 13, wobei das erste Protein die Aminosäuresequenz von SEQ ID NR: 9 umfasst.

15. T-Zelle nach einem der Ansprüche 1 bis 11, wobei die T-Zelle weiter ein zweites Protein exprimiert, das ein Tumorantigen oder ein Pathogenantigen erkennt.

16. T-Zelle nach Anspruch 15, wobei das zweite Protein ein chimärer Rezeptor ist.

17. T-Zelle nach Anspruch 16, wobei der chimäre Rezeptor ein CAR, ein chimärer T-Zell-Rezeptor oder ein TAC ist.

18. T-Zelle nach einem der Ansprüche 15 bis 17, wobei das zweite Protein eine extrazelluläre Domäne, eine Transmembrandomäne und eine intrazelluläre Signalisierungsdomäne umfasst.

19. T-Zelle nach einem der Ansprüche 15 bis 18, wobei das zweite Protein eine Antigenbindungsregion umfasst, die spezifisch BCMA erkennt.

20. T-Zelle nach Anspruch 19, wobei die Antigenbindungsregion, die spezifisch BCMA erkennt, die HCDR1-Sequenz von SEQ ID NR: 16, die HCDR2-Sequenz von SEQ ID NR: 17, die HCDR3-Sequenz von SEQ ID NR: 18, und die LCDR1-Sequenz von SEQ ID NR: 19, die LCDR2-Sequenz von SEQ ID NR: 20, die LCDR3-Sequenz von SEQ ID NR: 21 umfasst.

21. T-Zelle nach Anspruch 19, wobei die Antigenbindungsregion, die spezifisch BCMA erkennt, eine Sequenz der variablen Region der schweren Kette von SEQ ID NR: 22 und eine Sequenz der variablen Region der leichten Kette von SEQ ID NR: 23 umfasst.

22. T-Zelle nach Anspruch 19, wobei die Aminosäuresequenz des zweiten Proteins durch SEQ ID NR: 6 dargestellt ist.

23. T-Zelle nach einem der Ansprüche 12 bis 18, wobei das Tumorantigen umfasst: Thyreotropin-Rezeptor (TSHR); CD171; CS-1; C-Typ-Lektin-ähnliches Molekül-1; Gangliosid GD3; Tn-Antigen; CD19; CD20; CD 22; CD 30; CD70; CD123; CD138; CD33; CD44; CD44v7/8; CD38; CD44v6; B7H3 (CD276), B7H6; KIT(CD117); Interleukin-13-Rezeptor-Untereinheit a (IL-13Ra); Interleukin-11-Rezeptor a (IL-11Ra); Prostata-Stammzell-Antigen (PSCA); prostataspezifisches Membranantigen (PSMA); karzinoembryonales Antigen (CEA); NY-ESO-1; HIV-1 Gag; MART-1; gp100; Tyrosinase; Mesothelin; EpCAM; Protease-Serin-21 (PRSS21); Rezeptor für den vaskulären endothelialen Wachstumsfaktor, Rezeptor 2 für den vaskulären endothelialen Wachstumsfaktor (VEGFR2); Lewis-(Y)-Antigen; CD24; Rezeptor β für den von Blutplättchen abgeleiteten Wachstumsfaktor (PDGFR-β); stadienspezifisches embryonales Antigen-4 (SSEA-4); zelloberflächenassoziiertes Mucin 1 (MUC1), MUC6; Familie der Rezeptoren für den epidermalen Wachstumsfaktor und deren Mutanten (EGFR, EGFR2, ERBB3, ERBB4, EGFRvIII); neuronales Zelladhäsionsmolekül (NCAM); Carboanhydrase IX (CAIX); LMP2; Ephrin-A-Rezeptor 2 (EphA2); Fucosyl-GM1; Sialyl-Lewis-Adhäsionsmolekül (sLe); Gangliosid GM3; TGS5; hochmolekulares Melanom-assoziiertes Antigen (HMWMAA); O-Acetyl-GD2-Gangliosid (OAcGD2); Folsäurerezeptor; Tumor-Endothelmarker 1 (TEM1/CD248); mit Tumor-Endothelmarker 7 verwandtes Protein (TEM7R); Claudin 6, Claudin 18.2, Claudin 18.1; ASGPRI; CDH16; 5T4; 8H9; αvβ6-Integrin; B-Zell-Reifungsantigen (BCMA); CA9; K-Leichtkette (Kappa-Leichtkette); CSPG4; EGP2, EGP40; FAP; FAR; FBP; embryonaler AChR; HLA-A1, HLA-A2; MAGEA1, MAGE3; KDR; MCSP; NKG2D-Ligand; PSC1; ROR1; Sp17; SURVIVIN; TAG72; TEM1; Conectin; Tenascin; carcinoembryonale Variante der Tumornekrosezone; G-Protein-gekoppelten Rezeptor der Gruppe C, Mitglied D (GPRC5D); X-chromosomaler offener Leserahmen 61 (CXORF61); CD97; CD179a; anaplastische Lymphomkinase (ALK); Polysialinsäure; Plazentaspezifisches Protein 1 (PLAC1); Hexose-Rest von GloboH-Glykoceramid (GloboH); Brustdifferenzierungsantigen (NY-BR-1); Uroplakin 2 (UPK2); Hepatitis-A-Virus-Zellrezeptor 1 (HAVCR1); adrenergen Rezeptor β3 (ADRB3); Pannexin 3 (PANX3); G-Protein-gekoppelten Rezeptor 20 (GPR20); Lymphozytenantigen-6-Komplex-Locus K9 (LY6K); olfaktorischen Rezeptor 51E2 (OR51E2); TCRγ-Protein mit alternativem Leserahmen (TARP); Wilms-Tumor-Protein (WT1); ETS-Translokationsvarianten-Gen 6 (ETV6-AML); Spermienprotein 17 (SPA17); Mitglied 1A der X-Antigen-Familie (XAGE1); Angiogenin-bindender Zelloberflächenrezeptor 2 (Tie2); Melanom-Krebs-Hoden-Antigen 1 (MAD-CT-1); Melanom-Krebs-Hoden-Antigen 2 (MAD-CT-2); Fasverwandtes Antigen 1; p53-Mutante; humane Telomerase-Reverse-Transkriptase (hTERT); Sarkom-Translokationsbruchpunkt; Melanom-Apoptose-Inhibitor (ML-IAP); ERG (Transmembran-Serinprotease-2 (TMPRSS2)-ETS-Fusionsgen); N-Acetylglucosaminyltransferase V (NA17); Paired-Box-Protein Pax-3 (PAX3); Androgenrezeptor; Cyclin B1; V-myc-Aviäres-Myeloidose-Virus-Onkogen-Neuroblastom-abgeleitetes Homolog (MYCN); Ras-Homolog-Familienmitglied C (RhoC); Cytochrom P450 1B1 (CYP1B1); CCCTC-Bindungsfaktor (Zinkfingerprotein)-ähnlich (BORIS); von T-Zellen erkanntes Plattenepithelkarzinom-Antigen 3 (SART3); Paired-Box-Protein Pax-5 (PAX5); Proacrosin-Bindungsprotein sp32 (OYTES1); Lymphozyten-spezifische Protein-Tyrosinkinase (LCK); A-Kinase-Ankerprotein 4 (AKAP-4); Synovialsarkom-X-Bruchpunkt 2 (SSX2); CD79a; CD79b; CD72; Leukozyten-assoziierten Immunglobulin-ähnlichen Rezeptor 1 (LAIR1); Fc-Fragment-des-IgA-Rezeptors (FCAR); Leukozyten-Immunglobulin-ähnlichen Rezeptor-Unterfamilienmitglied 2 (LILRA2); CD300-Molekül-ähnliches Familienmitglied f (CD300LF); C-Typ-Lektin-Domänen-Familie-12-Mitglied A (CLEC12A); Knochenmark-Stromazell-Antigen 2 (BST2); EGF-ähnliches Modul enthaltender Mucin-ähnlichen Hormonrezeptor-ähnlichen Rezeptor 2 (EMR2); Lymphozytenantigen 75 (LY75); Glypican-3 (GPC3); Fc-Rezeptor-ähnliches Protein 5 (FCRL5); Immunglobulin-Lambda-ähnliches Polypeptid 1 (IGLL1).

24. T-Zelle nach einem der Ansprüche 12 bis 18, wobei das Pathogenantigen ausgewählt ist aus einer Gruppe, bestehend aus einem Virusantigen, einem Bakterienantigen, einem Pilzantigen, einem Protozoenantigen und einem Parasitenantigen, wobei das Virusantigen optional ausgewählt ist aus einer Gruppe, bestehend aus einem Zytomegalievirus-Antigen, einem Epstein-Barr-Virus-Antigen, einem Humanen-Immundefizienz-Virus-Antigen und einem Influenzavirus-Antigen.

25. T-Zelle nach einem der Ansprüche 9 bis 24, wobei das HLA-1-Gen unter Verwendung einer sgRNA, deren Sequenz durch SEQ ID NR: 8 dargestellt ist, stillgelegt ist und/oder das TCR-Gen unter Verwendung einer sgRNA, deren Sequenz durch SEQ ID NR: 7 dargestellt ist, stillgelegt ist.

26. T-Zelle nach einem der Ansprüche 1 bis 25, wobei die T-Zelle eine autologe T-Zelle oder eine allogene T-Zelle ist.

27. T-Zelle nach einem der Ansprüche 1 bis 26 zur Verwendung in einem Verfahren zur Verhinderung oder Regulierung einer immunologischen Transplantationsabstoßung.

28. T-Zelle nach einem der Ansprüche 1 bis 26 zur Verwendung in einem Verfahren zur Verhinderung oder Regulierung des Angriffs von NK-Zellen auf exogene Zellen.

29. T-Zelle zur Verwendung nach Anspruch 28, wobei die exogenen Zellen (i) T-Zellen, NK-T-Zellen oder Stammzellen; oder (ii) gentechnisch veränderte T-Zellen, NK-T-Zellen oder Stammzellen sind.

## Revendications

1. Lymphocyte T résistant au rejet de greffe par le système immunitaire,
dans lequel le lymphocyte T exprime une première protéine qui est un récepteur chimérique reconnaissant NKG2A sur des cellules NK d'un hôte ;
dans lequel le récepteur chimérique comprend un domaine extracellulaire présentant une région de liaison à l'antigène reconnaissant NKG2A, un domaine transmembranaire et un domaine de signalisation intracellulaire ; et
dans lequel, dans le lymphocyte T, le gène HLA-I et le gène TCR endogène sont mis sous silence.

2. Lymphocyte T selon la revendication 1, dans lequel le récepteur chimérique est un récepteur antigénique chimérique (CAR), un récepteur chimérique des lymphocytes T ou un coupleur antigénique de lymphocyte (TAC).

3. Lymphocyte T selon la revendication 1 ou revendication 2, dans lequel la région de liaison à l'antigène est choisie dans le groupe consistant en scFv, Fv, Fab, Fab', Fab'-SH, F(ab')₂, un fragment à domaine unique et un ligand naturel engageant son récepteur homologue.

4. Lymphocyte T selon l'une quelconque des revendications 1 à 3, dans lequel la première protéine comprend une région de liaison à l'antigène comprenant une séquence HCDR1 de SEQ ID NO : 10, une séquence HCDR2 de SEQ ID NO : 11, une séquence HCDR3 de SEQ ID NO : 12 ; et une séquence LCDR1 de SEQ ID NO : 13, une séquence LCDR2 de SEQ ID NO : 14, une séquence LCDR3 de SEQ ID NO : 15.

5. Lymphocyte T selon la revendication 4, dans lequel la première protéine comprend une région de liaison à l'antigène comprenant une séquence de régions variables de chaîne lourde de SEQ ID NO : 1, ou une séquence de régions variables de chaîne légère de SEQ ID NO : 2.

6. Lymphocyte T selon la revendication 4 ou revendication 5, dans lequel la séquence d'acides aminés de la première protéine est représentée par SEQ ID NO : 5.

7. Lymphocyte T selon l'une quelconque des revendications 1 à 6, dans lequel le gène HLA-I est un ou plusieurs gènes choisis dans le groupe consistant en : HLA-A, HLA-B, HLA-C et B2M.

8. Lymphocyte T selon la revendication 7, dans lequel le gène HLA-I est B2M.

9. Lymphocyte T selon l'une quelconque des revendications 1 à 8, dans lequel le gène HLA-I et le gène TCR endogène sont mis sous silence par une technologie de modification génétique.

10. Lymphocyte T selon la revendication 9, dans lequel la technologie de modification génétique est choisie dans le groupe consistant en : technologie CRISPR/Cas9, technologie de nucléase à doigt de zinc (ZFN) artificielle, technologie d'effecteur de type activateur de transcription (TALE) et technologie TALE- CRISPR/Cas9.

11. Lymphocyte T selon la revendication 10, dans lequel la technologie de modification génétique est la technologie CRISPR/Cas9.

12. Lymphocyte T selon l'une quelconque des revendications 1 à 11, dans lequel la première protéine comprend en outre une région de liaison à l'antigène reconnaissant un antigène tumoral ou un antigène pathogène.

13. Lymphocyte T selon la revendication 12, dans lequel la région de liaison à l'antigène reconnaissant NKG2A de l'hôte et la région de liaison à l'antigène reconnaissant un antigène tumoral ou un antigène pathogène sont reliées par un peptide lieur.

14. Lymphocyte T selon la revendication 12 ou revendication 13, dans lequel la première protéine comprend la séquence d'acides aminés de SEQ ID NO : 9.

15. Lymphocyte T selon l'une quelconque des revendications 1 à 11, dans lequel le lymphocyte T exprime en outre une deuxième protéine reconnaissant un antigène tumoral ou un antigène pathogène.

16. Lymphocyte T selon la revendication 15, dans lequel la deuxième protéine est un récepteur chimérique.

17. Lymphocyte T selon la revendication 16, dans lequel le récepteur chimérique est un CAR, un récepteur chimérique des lymphocytes T ou un TAC.

18. Lymphocyte T selon l'une quelconque des revendications 15 à 17, dans lequel la deuxième protéine comprend un domaine extracellulaire, un domaine transmembranaire et un domaine de signalisation intracellulaire.

19. Lymphocyte T selon l'une quelconque des revendications 15 à 18, dans lequel la deuxième protéine comprend une région de liaison à l'antigène reconnaissant spécifiquement BCMA.

20. Lymphocyte T selon la revendication 19, dans lequel la région de liaison à l'antigène reconnaissant spécifiquement BCMA comprend une séquence HCDR1 de SEQ ID NO : 16, une séquence HCDR2 de SEQ ID NO : 17, une séquence HCDR3 de SEQ ID NO : 18 et une séquence LCDR1 de SEQ ID NO : 19, une séquence LCDR2 de SEQ ID NO : 20, une séquence LCDR3 de SEQ ID NO : 21.

21. Lymphocyte T selon la revendication 19, dans lequel la région de liaison à l'antigène reconnaissant spécifiquement BCMA comprend une séquence de régions variables de chaîne lourde de SEQ ID NO : 22 et une séquence de régions variables de chaîne légère de SEQ ID NO : 23.

22. Lymphocyte T selon la revendication 19, dans lequel la séquence d'acides aminés de la deuxième protéine est représentée par SEQ ID NO :6.

23. Lymphocyte T selon l'une quelconque des revendications 12 à 18, dans lequel l'antigène tumoral comprend le récepteur de l'hormone de stimulation de la thyroïde (TSHR) ; CD171 ; CS-1 ; molécule-1 analogue à lectine de type-C ; ganglioside GD3 ; antigène Tn ; CD19 ; CD20 ; CD 22 ; CD 30 ; CD70 ; CD123 ; CD138 ; CD33 ; CD44 ; CD44v7/8 ; CD38 ; CD44v6 ; B7H3(CD276), B7H6 ; KIT(CD 117) ; sous-unité du récepteur a de l'interleukine 13 (IL-13Ra) ; récepteur a de l'interleukine 11 (IL-11Ra) ; antigène de cellule souche prostatique (PSCA) ; antigène membranaire spécifique de la prostate (PSMA) ; antigène carcinoembryonnaire (CEA) ; NY-ESO-1 ; HIV-1 Gag ; MART- 1 ; gp100 ; tyrosinase ; mésothéline ; EpCAM ; protéase sérine 21 (PRSS21) ; récepteur du facteur de croissance endothélial vasculaire, récepteur 2 du facteur de croissance endothélial vasculaire (VEGFR2) ; antigène Lewis (Y) ; CD24 ; récepteur β du facteur de croissance dérivé des plaquettes (PDGFR-β) ; antigène embryonnaire spécifique au stade-4 (SSEA-4) ; mucine 1 associée à la surface cellulaire (MUC1), MUC6 ; famille du récepteur du facteur de croissance épidermique et ses mutants (EGFR, EGFR2, ERBB3, ERBB4, EGFRvIII) ; molécule d'adhérence cellulaire neurale (NCAM) ; anhydrase carbonique IX (CAIX) ; LMP2 ; récepteur 2 de l'éphrine A (EphA2) ; fucosyl GM1 ; molécule d'adhérence sialyl Lewis (sLe) ; ganglioside GM3 ; TGS5 ; antigène associé au mélanome de haut poids moléculaire (HMWMAA) ; ganglioside GD2 O-acétylé (OAcGD2) ; récepteur de l'acide folique ; marqueur 1 endothélial vasculaire tumoral (TEM1/CD248) ; apparenté au marqueur 7 endothélial vasculaire tumoral (TEM7R) ; Claudine 6, Claudine 18.2, Claudine 18.1 ; ASGPRI ; CDH16 ; 5T4 ; 8H9 ; intégrine αvβ6 ; antigène de maturation des lymphocytes B (BCMA) ; CA9 ; chaîne légère K (chaîne légère kappa) ; CSPG4 ; EGP2, EGP40 ; FAP ; FAR ; FBP ; AchR embryonnaire ; HLA-A1, HLA-A2 ; MAGEA1, MAGE3 ; KDR ; MCSP ; ligand NKG2D ; PSC1 ; ROR1 ; Sp17 ; SURVIVINE ; TAG72 ; TEM1 ; connectine ; ténascine ; variant carcinoembryonnaire de zone de nécrose tumorale ; membre D de groupe 5 de récepteur C couplé à la protéine (GPRC5D) ; cadre ouvert de lecture 61 du chromosome X (CXORF61) ; CD97 ; CD179a ; kinase du lymphome anaplasique (ALK) ; acide polysialique ; spécifique du placenta 1 (PLAC1) ; fraction hexose de glycocéramide globoH (GloboH) ; antigène de différenciation mammaire (NY-BR-1) ; uroplakine 2 (UPK2) ; récepteur 1 de cellule du virus de l'hépatite A (HAVCR1) ; récepteur β3 adrénergique (ADRB3) ; pannexine 3 (PANX3) ; récepteur 20 couplé à la protéine G (GPR20) ; locus K9 du complexe d'antigène lymphocytaire 6 (LY6K) ; récepteur olfactif 51E2 (OR51E2) ; protéine de cadre de lecture alternatif TCRy (TARP) ; protéine tumorale de Wilms (WT1) ; gène variant de translocation 6 ETS (ETV6-AML) ; protéine spermatique 17 (SPA17) ; membre 1A de la famille d'antigène X (XAGE1) ; récepteur 2 de surface cellulaire de liaison à l'angiogénine (Tie2) ; antigène testicule-cancer mélanome-1 (MAD-CT-1) ; antigène testicule-cancer mélanome-2 (MAD-CT-2) ; antigène 1 lié à Fas ; mutant p53 ; transcriptase inverse de la télomérase humaine (hTERT) ; point de cassure de translocation de sarcome ; inhibiteur de l'apoptose du mélanome (ML-IAP) ; ERG (gène de fusion ETS de la protéase transmembranaire sérine 2 (TMPRSS2)) ; N-acétylglucosaminyl transférase V (NA17) ; protéine boîte appariée Pax-3 (PAX3) ; récepteur des androgènes ; cycline B1 ; homologue dérivé de neuroblastome de l'oncogène V-myc du virus de la myéloïdose aviaire (MYCN) ; membre C de la famille des homologues de Ras (RhoC) ; cytochrome P450 1B1 (CYP1B1) ; de type facteur de liaison CCCTC (protéine à doigt de zinc) (BORIS) ; antigène 3 de carcinome épidermoïde (SART3) reconnu par des lymphocytes T ; protéine boîte appariée Pax-5 (PAX5) ; protéine de liaison à la proacrosine sp32 (OYTES1) ; protéine tyrosine kinase spécifique des lymphocytes (LCK) ; protéine 4 d'ancrage de kinase A (AKAP-4) ; point de cassure 2 du sarcome synovial X (SSX2) ; CD79a ; CD79b ; CD72 ; récepteur 1 de type immunoglobuline associé aux leucocytes (LAIR1) ; fragment Fe du récepteur de l'IgA (FCAR) ; membre 2 de la sous-famille de récepteurs de type immunoglobuline leucocytaire (LILRA2) ; membre f de la famille de type moléculaire CD300 (CD300LF) ; membre A de la famille 12 à domaine lectine de type C (CLEC12A) ; antigène 2 des cellules stromales de la moelle osseuse (BST2) ; contenant un module de type EGF, de type récepteur hormonal de type mucine 2 (EMR2) ; antigène lymphocytaire 75 (LY75) ; glypicane-3 (GPC3) ; de type récepteur Fc 5 (FCRL5) ; polypeptide 1 de type lambda d'immunoglobuline (IGLL1).

24. Lymphocyte T selon l'une quelconque des revendications 12 à 18, dans lequel l'antigène pathogène est choisi dans un groupe consistant en un antigène viral, un antigène bactérien, un antigène fongique, un antigène protozoaire et un antigène parasitaire, facultativement dans lequel l'antigène viral est choisi dans un groupe consistant en un antigène de cytomégalovirus, un antigène du virus d'Epstein-Barr, un antigène du virus de l'immunodéficience humaine et un antigène du virus de la grippe.

25. Lymphocyte T selon l'une quelconque des revendications 9 à 24, dans lequel le gène HLA-1 est mis sous silence en utilisant un ARNsg, dont la séquence est représentée par SEQ ID NO : 8, et/ou le gène TCR est mis sous silence en utilisant un ARNsg, dont la séquence est représentée par SEQ ID NO : 7.

26. Lymphocyte T selon l'une quelconque des revendications 1 à 25, dans lequel le lymphocyte T est un lymphocyte T autologue ou un lymphocyte T allogénique.

27. Lymphocyte T tel que défini dans l'une quelconque des revendications 1 à 26 pour une utilisation dans un procédé de prévention ou de régulation du rejet de greffe par le système immunitaire.

28. Lymphocyte T tel que défini dans l'une quelconque des revendications 1 à 26 pour une utilisation dans un procédé de prévention ou de régulation de l'attaque de cellule NK sur des cellules exogènes.

29. Lymphocyte T pour utilisation de la revendication 28, dans lequel les cellules exogènes sont (i) des lymphocytes T, des cellules NK T ou des cellules souches ; ou (ii) des lymphocytes T, des cellules NK T ou des cellules souches modifiés.
